# EUROPEAN PATENT SPECIFICATION

(11) **EP 1 064 296 B1**
(45) Date of publication and mention of the grant of the patent: **19.10.2005**
(21) Application number: 99911400.2
(22) Date of filing: 17.03.1999
(51) Int. Cl.: C07H 21/04, C12N 1/14, C12N 1/20, C12N 5/00, C12N 9/12, C12N 15/00, C12P 19/34, C12Q 1/68

(54) **THERMOSTABLE DNA POLYMERASE FROM THERMOANAEROBACTER THERMOHYDROSULFURICUS**
TEMPERATURSTABILE DNA POLYMERASE AUS THERMOANAEROBACTER THERMOHYDROSULFURICUS
POLYMERASE D'ADN THERMOSTABLE PROVENANT DU THERMOANAEROBACTER THERMOHYDROSULFURICUS

(30) Priority: 18.03.1998 US 44106
(43) Date of publication of application: 03.01.2001
(73) Proprietor: Amersham Biosciences Corp., Piscataway, N.J. 08855-1327 (US)
(72) Inventor: MAMONE, Joseph, A., Parma, OH 44134 (US); DAVIS, Maria, Twinsburg, OH 44087 (US); SHA, Dan, Euclid, OH 44117 (US)
(74) Representative: Rollins, Anthony John
(86) International application number: PCT/US1999/005612
(87) International publication number: WO 1999/047539

(56) References cited:
- EP-A- 0 479 158
- WO-A-91/09944
- WO-A-91/09950
- WO-A1-97/21821
- US-A- 5 407 800
- US-A- 5 744 312
- PERLER F B ET AL: "THERMOSTABLE DNA POLYMERASES" , ADVANCES IN PROTEIN CHEMISTRY, ACADEMIC PRESS, NEW YORK, NY, US, VOL. 48, PAGE(S) 377-435 XP000654858 ISSN: 0065-3233 * page 381 - page 385 * * page 415 - page 421 * * page 393 - page 415 *

## Description

This application claims priority to Mamone et al., U.S. Serial No. 08/766, 014 filed December 13, 1996, entitled "Thermostable DNA Polymerase From Thermoanaerobacter Thermohydrosulfuricus" which claims priority to U.S. Serial No. 60/008,688, filed December 15, 1995, entitled "Thermostable DNA Polymerase."

### Background of the Invention

The present invention relates to novel thermostable DNA polymerases obtainable from the thermophilic anaerobe Thermoanaerobacter thermohydrosulfuricus, to certain deletants and mutants of this enzyme, to genes and vectors encoding the wild type and mutant polymerases and their use in strand displacement activity, polymerase chain reaction, DNA sequencing and as reverse transcriptases. The following is a discussion of the relevant art, none of which is admitted to be prior art to the appended claims. DNA polymerases are a family of enzymes involved in DNA repair and replication. DNA polymerases have been isolated from E.coli (e.g. E.coli DNA polymerase I and the Klenow fragment thereof) and T4 DNA polymerase and more recently thermostable DNA polymerases have been isolated (e.g. from T. aquaticus, US Patent 4,889,818, and from T. litoralis). Thermostable DNA polymerases have been suggested (US Patent 4,683,195) for use in amplifying existing nucleic acid sequences in amounts that are large compared to that originally present. The polymerase chain reaction (PCR) and strand displacement amplification (SDA) are two methods of amplifying nucleic acid sequences.

PCR is based on the hybridization of oligonucleotide primers to specific sequences on opposite strands of the target DNA molecule, and subsequent extension of these primers with a DNA polymerase to generate two new strands of DNA which themselves can serve as a template for a further round of hybridization and extension. In PCR reactions the product of one cycle serves as the template for the next cycle such that at each repeat of the cycle the amount of the specific sequence present in the reaction doubles leading to an exponential amplification process.

PCR relies on a process of temperature cycling to promote the amplification reaction. This temperature cycling is necessary to separate the strands of DNA formed in one cycle of the reaction to allow hybridization of the oligonucleotides required to initiate the next cycle. DNA strand separation is usually achieved by melting the DNA at temperatures in the range 90-100°C followed by cooling to a lower temperature to allow oligonucleotide hybridization followed by extension or ligation, depending on the reaction process. This cycling process may be repeated 20-50 times, again depending on the process and the degree of amplification required.

Temperature cycling is normally achieved by the use of specialized equipment, commonly termed thermocyclers, which can operate by a wide variety of mechanical, electrical or hydraulic means, but serve a common purpose in heating and cooling a small container or a number of such containers in which the amplification reaction is performed.

In order for the amplification reactions to proceed with the desired efficiency and specificity it is necessary to perform the temperature cycling process within strictly defined and reproducible limits of temperature and time. Failure of the temperature cycling apparatus to achieve the required conditions will result in the partial or total failure of the amplification reaction. These strict requirements on time and temperature of cycling can impose severe restrictions if the handling of large numbers of reactions is required. If it is desired to perform several hundred or more reactions simultaneously use of conventional thermocyclers would be extremely expensive in terms of the capital investment required in equipment, and would in any case be prone to variations between individual thermocyclers. Alternative solutions for large scale use have been constructed based on fan assisted oven or multiple water baths, but such solutions are inevitably cumbersome in use and in any case still require the user to take the normal stringent steps to avoid evaporation which become extremely difficult to apply to a large number of cycles.

In reverse transcription/ polymerase chain reaction (RT/PCR), a DNA primer is hybridized to a strand of the target RNA molecule, and subsequent extension of this primer with a reverse transcriptase generates a new strand of DNA which can serve as a template for PCR. Preparation of the DNA template is preferably carried out at an elevated temperature to avoid early termination of the reverse transcriptase reaction caused by RNA secondary structure. There is a lack of efficient reverse transcriptases that act at elevated temperatures, e.g. above 50 °C.

SDA differs from PCR in being an isothermal amplification process, i.e. all reactions occur at the same temperature without the need for elevated temperature to melt DNA strands. This is made possible by adoption of a reaction scheme which uses the ability of certain DNA polymerases when extending along a DNA template strand to displace any DNA molecules already hybridized to the template. In SDA this strand displacement is used to separate the double stranded DNA produced during the reaction process and hence to maintain continuous amplification of the target DNA sequence (Walker, G.T., Little, M.C., Nadeau, J.G. and Shank D.D. (1992) *Proc*. *Natl. Acad. Sci. USA* 89:392-396). SDA is therefore in principle more suited to use with large numbers of samples than PCR as the isothermal process, which is performed at temperatures of 37 OC to 60 OC, does not require stringent precautions to be taken to avoid evaporation and can be performed with simple temperature control equipment, for example in a standard laboratory incubator.

DNA polymerases, e.g. Sequenase, Klenow, Taq, etc, have also been extensively used in DNA sequencing, see for example "Molecular Cloning: A Laboratory Manual" (Sambrook, Fritsch, and Maniatis, 2nd edition, Cold Spring Harbor laboratory Press, 1989).

### Summary of the Invention

The present invention provides a thermostable DNA polymerase from Thermoanaerobacter thermohydrosulfuricus.

This enzyme is useful for procedures requiring strand-displacing DNA synthesis such as SDA, for DNA sequencing, reverse transcription and polymerase chain reaction. Included within the scope of the present invention are various mutants (deletion and substitution) that retain thermostability and the ability to replicate DNA with substantially the same efficiency as the native Thermoanaerobacter thermohydrosulfuricus polymerase.

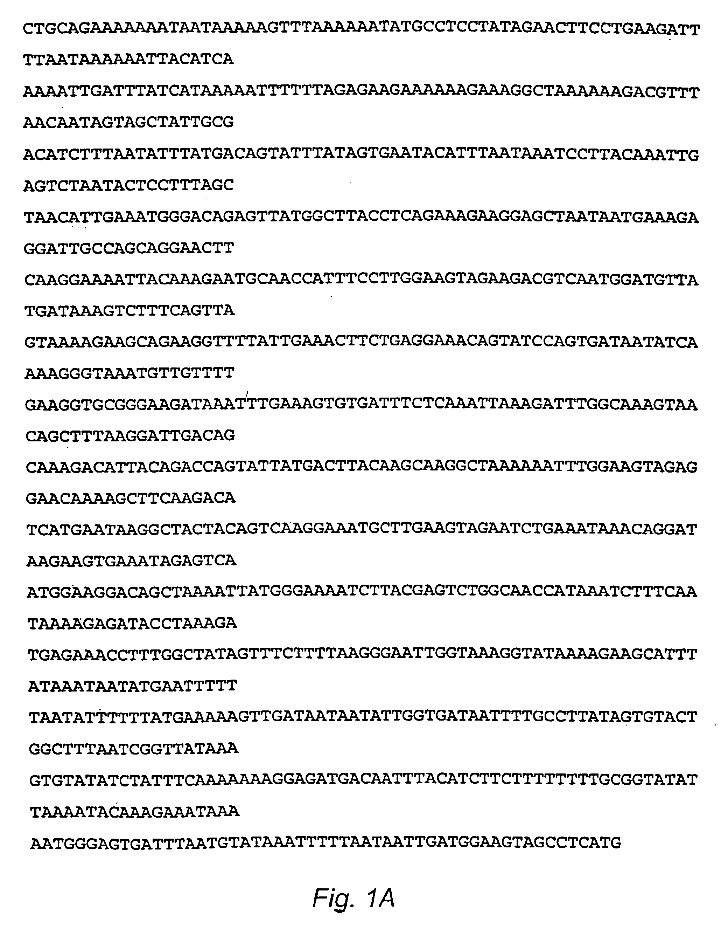

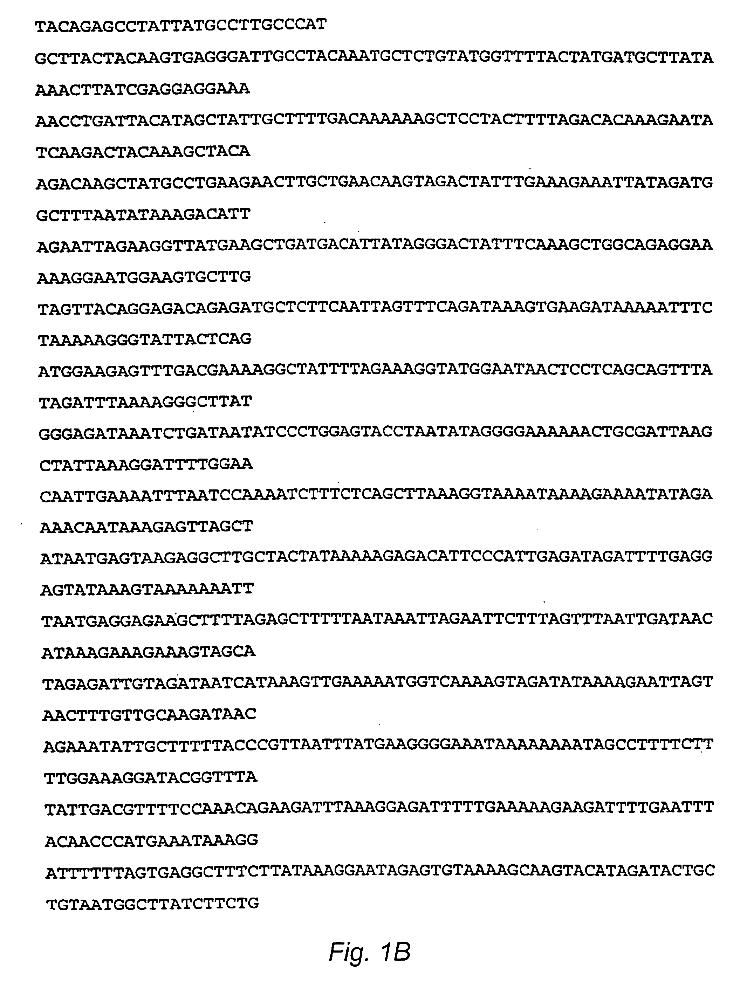

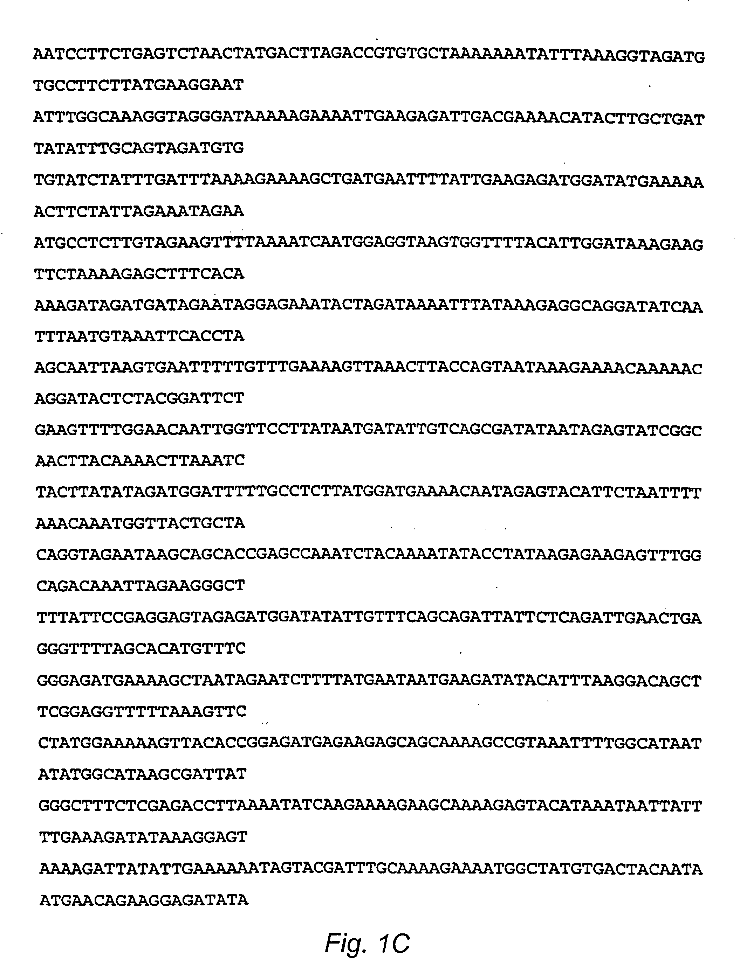

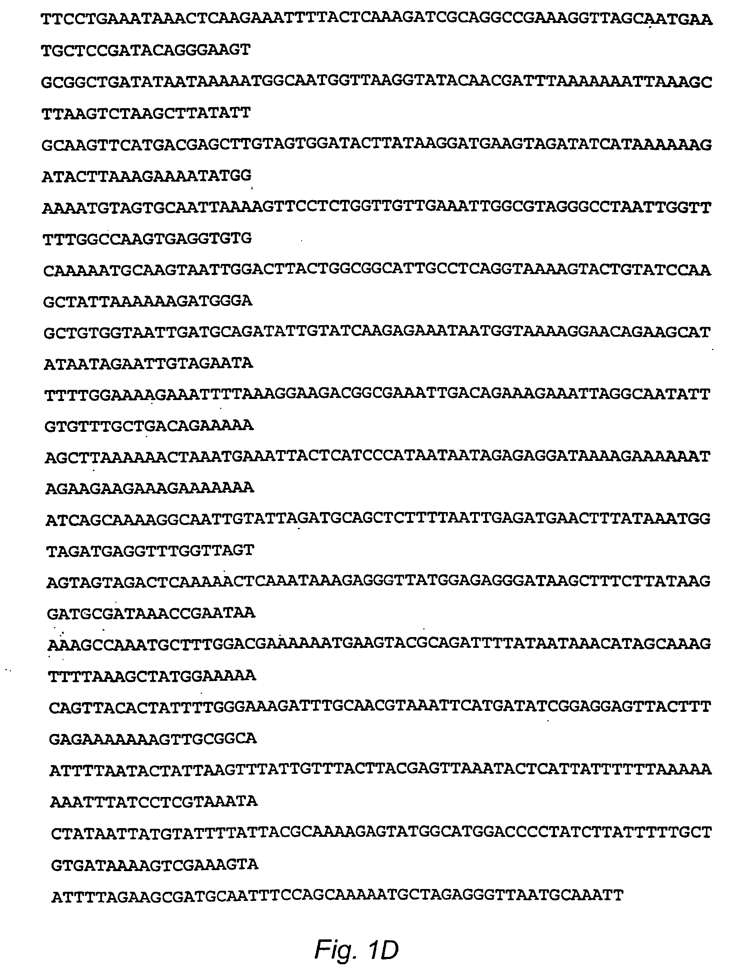

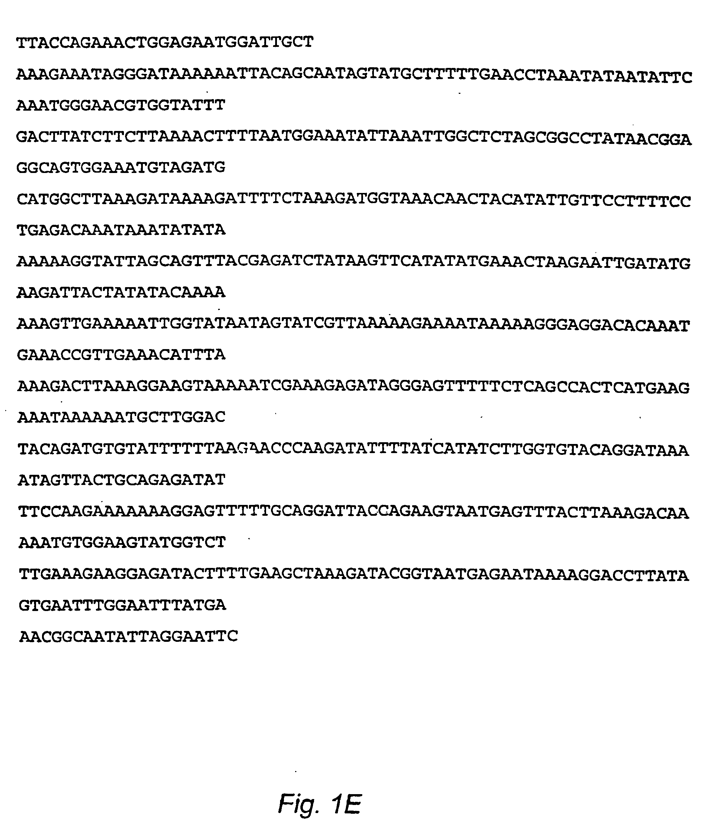

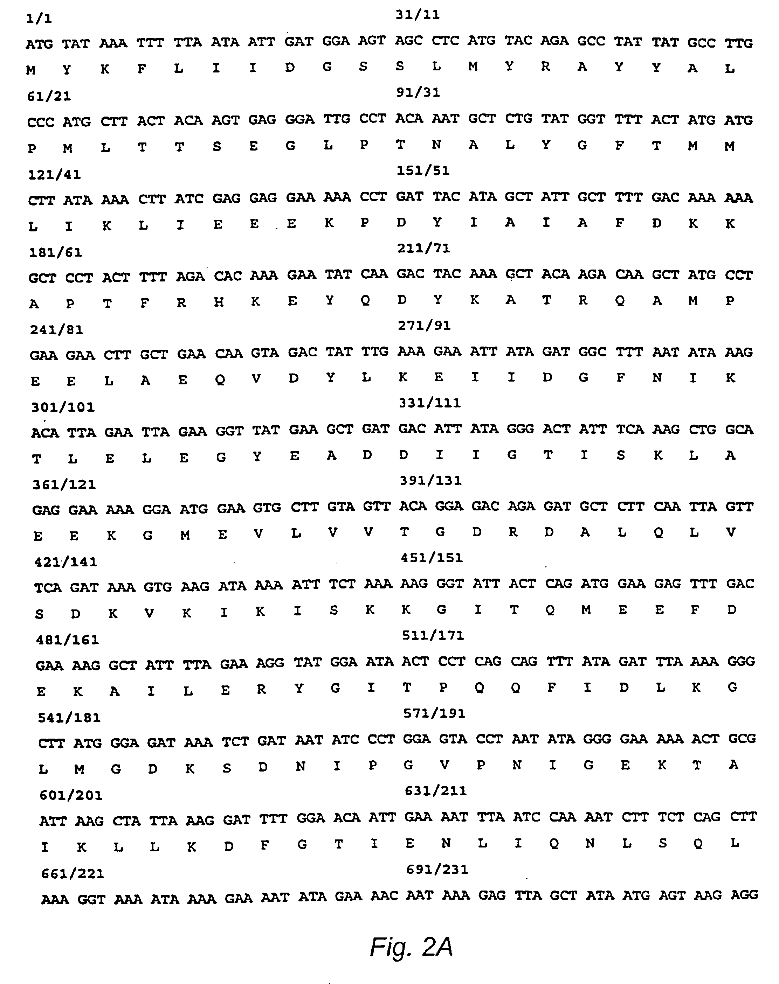

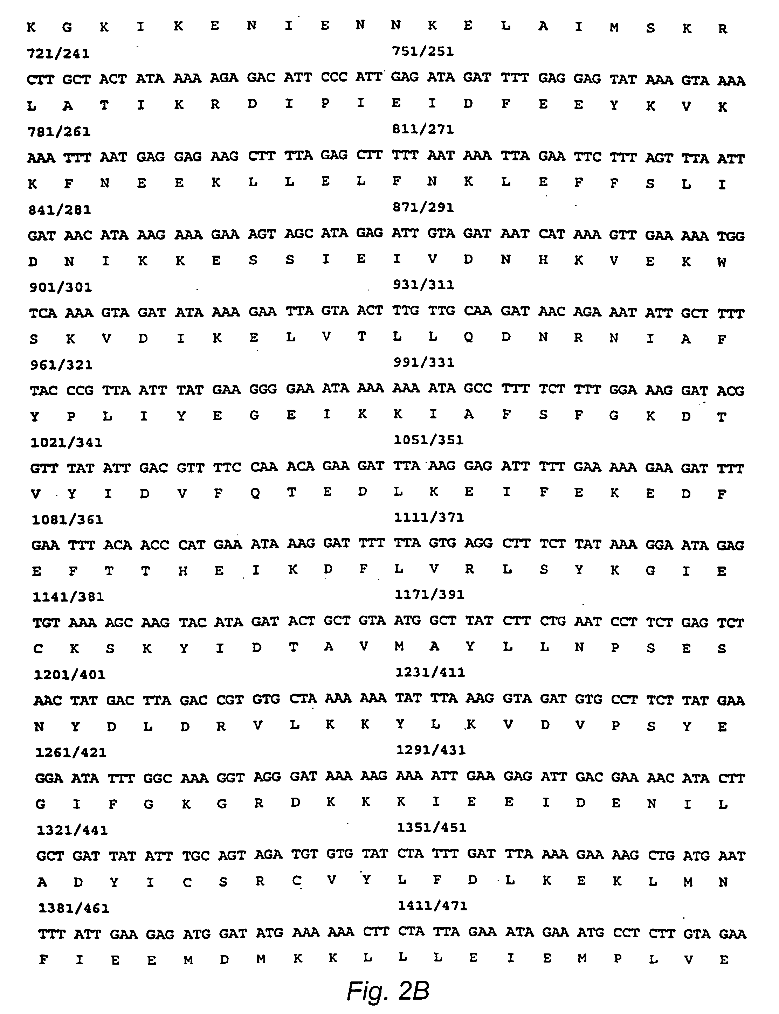

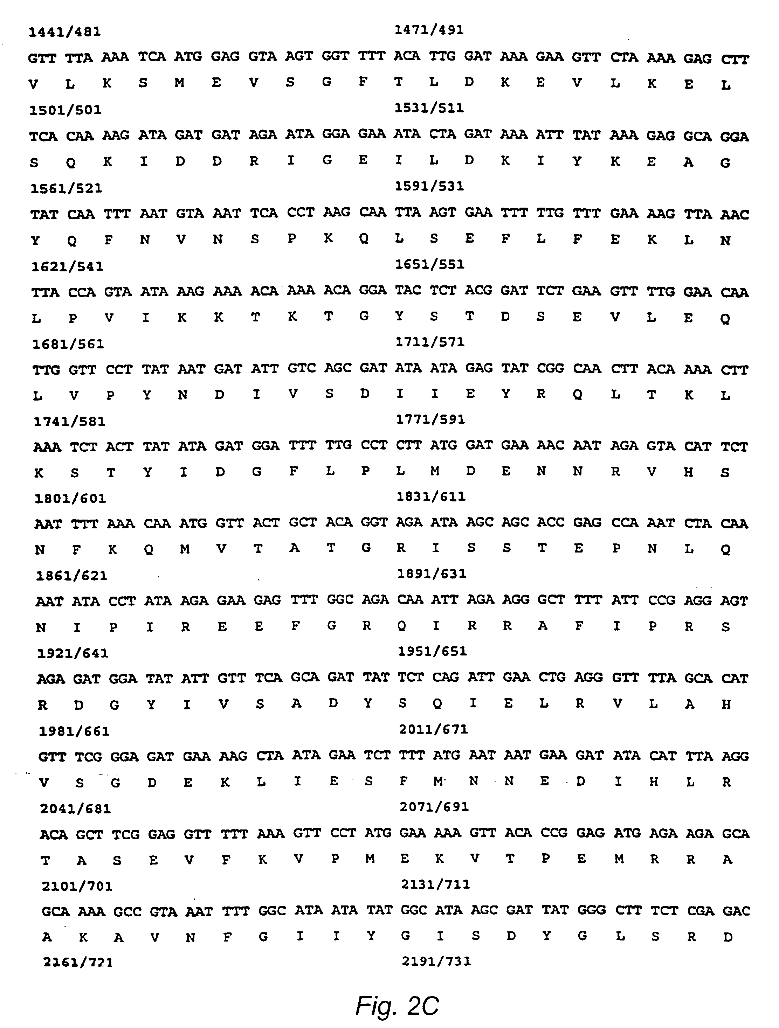

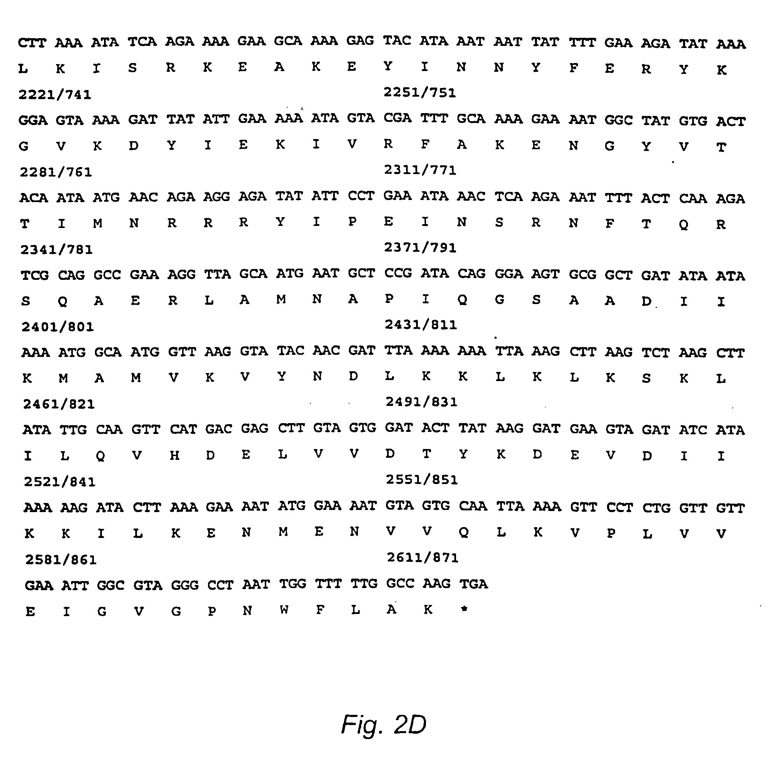

In a first aspect, the present invention provides a purified DNA polymerase or fragment thereof having the DNA polymerase activity of Thermoanaerobacter thermohydrosulfuricus of Seq, ID. No. 2 and having the exonuclease activity removed by replacing the aspartic acid residue at position 8 with an alanine residue and having at least 80% amino acid homology, preferably at least 900-. homology, to at least a contiguous amino acid sequence shown in Fig. 2 A-D (SEQ. ID. NO. 2). Fig. 2A-D represents the translation of an open reading frame spanning nucleotides 1056-3674 of genomic DNA encoding a thermostable DNA polymerase from Thermoanaerobacter thermohydrosulfuricus (Fig. 1A-E) (SEQ. ID. NO. 1) potentially encoding the native polymerase.

When used herein, the term amino acid homology means the amino acid identity of the parent enzyme or conservative amino acid changes thereto. The DNA polymerase can be encoded by a full-length nucleic acid sequence or any portion of the full-length nucleic acid sequence, so long as a functional activity of the polypeptide is retained. The amino acid sequence will be substantially similar to the sequence shown in Fig. 2A-D, or fragments thereof. A sequence that is substantially similar will preferably have at least 80% identity (more preferably at least 90% and most preferably 98-100%) to the sequence of Fig. 2A-D.

By "identity" is meant a property of sequences that measures their similarity or relationship. Identity is measured by dividing the number of identical residues by the total number of residues and multiplying the product by 100. Thus, two copies of exactly the same sequence have 100% identity, but sequences that are less highly conserved, and have deletions, additions, or replacements, may have a lower degree of identity. Those skilled in the art will recognize that several computer programs are available for determining sequence identity.

The purified enzyme of the present invention has a molecular weight of approximately 90,000 daltons when measured on SDS-PAGE. It possesses a 5'-3' exonuclease activity. The temperature optimum of DNA synthesis is near 75 °C under assay conditions. The optimum magnesium ion and manganese ion concentrations for DNA synthesis are 1 mM and 0.5 mM respectively.

The term thermostable polymerase means an enzyme which is stable to heat (and heat resistant) and is suitable for use in SDA and/or sequencing at an elevated temperature, for example 70 °C. The thermostable enzyme herein must satisfy a single criterion to be effective for the amplification reaction, i.e., the enzyme must not become irreversibly denatured (inactivated) when subjected to the elevated temperatures for the time necessary to effect amplification. Irreversible denaturation for purposes herein refers to permanent and complete loss of enzymatic activity. Preferably, the enzyme will not become irreversibly denatured at about 70 °C but may become denatured at higher temperatures. The thermostable enzyme herein preferably has an optimum temperature at which it functions that is higher than about 40 °C, which is the temperature below which hybridization of primer to template is promoted, although, depending on (1) salt concentration and composition and (2) composition and length of primer, hybridization can occur at higher temperature (e.g., 45-70 °C). The higher the temperature optimum for the enzyme, the greater the specificity and/or selectivity of the primer-directed extension process. However, enzymes that are active below 40 °C, e.g., at 37 °C, are also within the scope of this invention provided they are heat stable. Preferably, the optimum temperature ranges from about 50 to 80 °C, more preferably 50-70 °C.

When used herein, the term a DNA polymerase or fragment thereof having the DNA polymerase activity of *Thermoanaerobacter thermohydrosulfuricus* means a DNA polymerase or fragment thereof (as hereinafter defined) which has the ability to replicate DNA with substantially the same efficiency as the enzyme encoded by the SEQ. ID. NO. 1. By substantially the same efficiency is meant at least 80% and preferably at least 90% of the efficiency of the enzyme encoded by SEQ. ID. NO. 1 to incorporate deoxynucleotides.

The invention also encompasses a stable enzyme composition which comprises a purified thermostable DNA polymerase from *Thermoanaerobacter thermohydrosulfuricus* of Seq. ID.No.2 and having the exonuclease activity removed by replacing the aspartic acid residue at position 8 with an alanine residue in a buffer.

The DNA polymerases of the present invention are preferably in a purified form. By purified is meant that the DNA polymerase is isolated from a majority of host cell proteins normally associated with it; preferably the polymerase is at least 10% (w/w), e.g. at least 50% (w/w), of the protein of a preparation, even more preferably it is provided as a homogeneous preparation, e.g.. homogeneous solution. Preferably the DNA polymerase is a single polypeptide on an SDS polyacrylamide gel.

Buffers around neutral pH (5-9) such as 5-100 mM TrisHCl, phosphate or MES are suitable for use in the current invention.

It has been found that the entire amino acid sequence of the polymerase is not required for enzymatic activity. Thus, for example, the exonuclease domain of the enzyme has been deleted to give an enzyme of molecular weight of approximately 64,000 daltons when measured on SDS-PAGE which retains enzyme activity and also has reverse transcriptase activity making it useful for making cDNA. This exonuclease-free enzyme is analogous to the Klenow fragment of E. coli DNA polymerase I - Thus, the present invention also provides fragments of the polymerase which retain the DNA polymerase activity of *Thermoanaerobacter thermohydrosulfuricus* but have one or more amino acids deleted, preferably from the amino-terminus, while still having at least 80% amino acid homology to at least a 40 contiguous amino acid sequence shown in Fig. 2A-D (SEQ. ID. NO. 2).

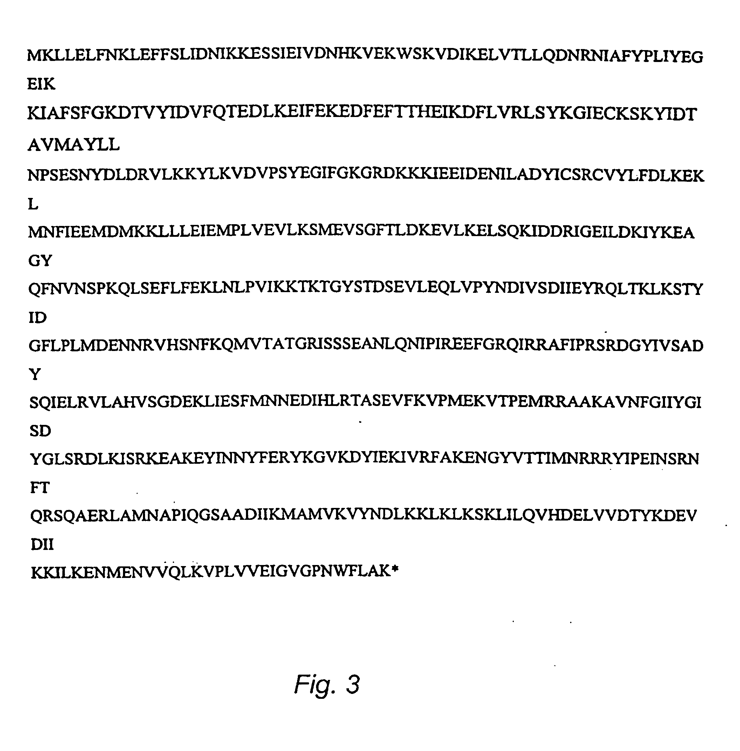

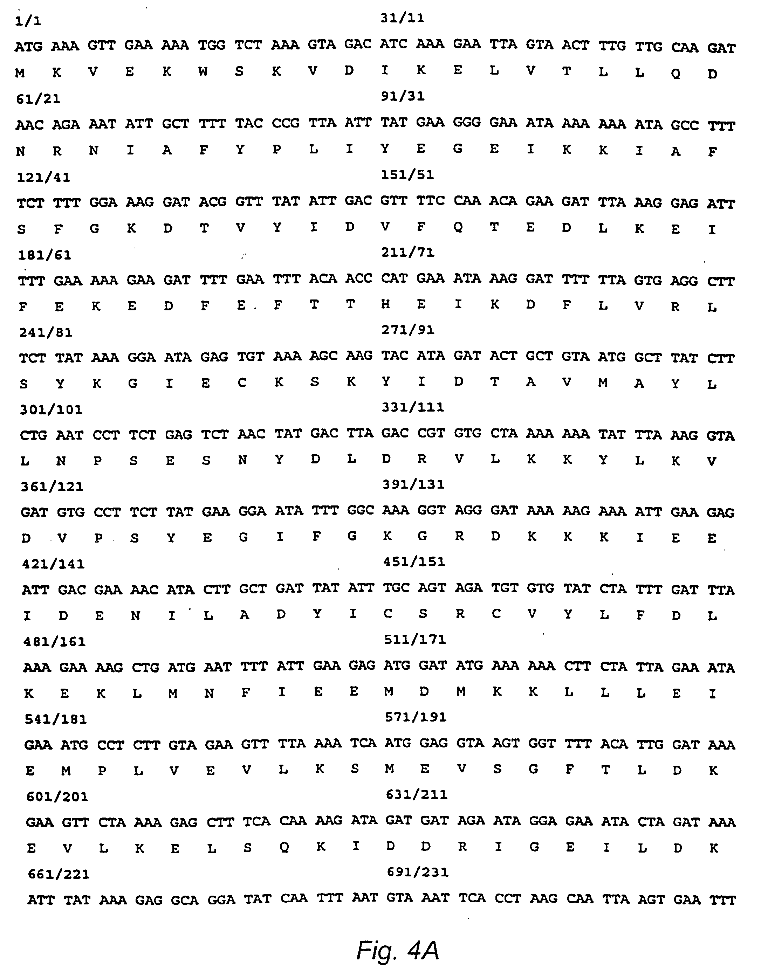

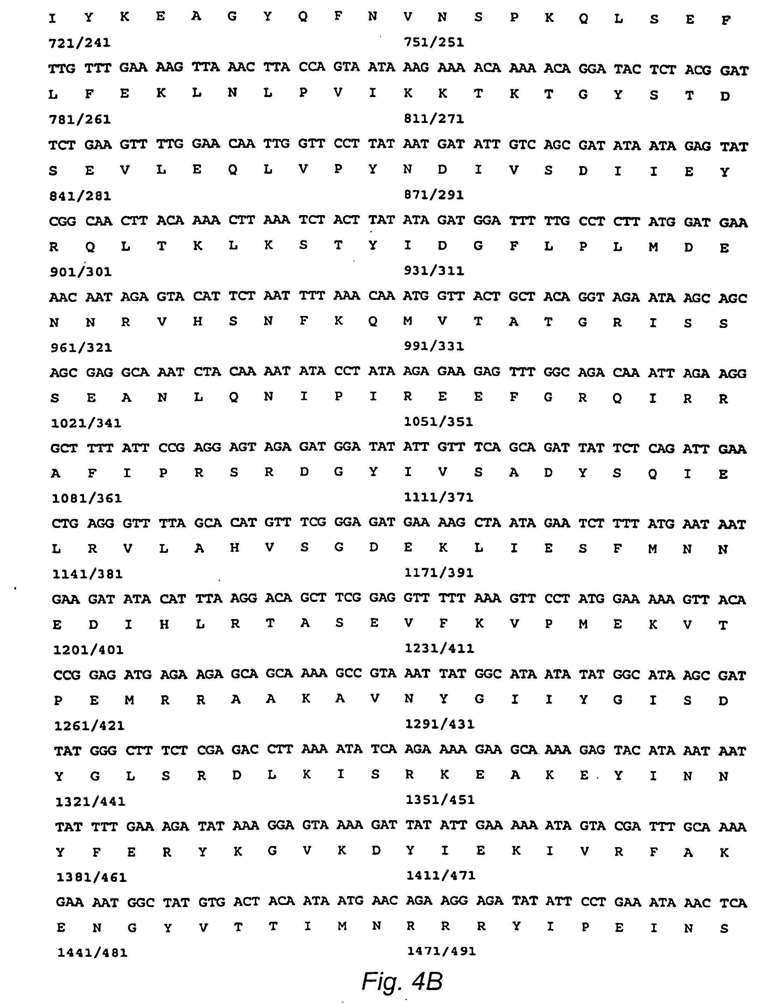

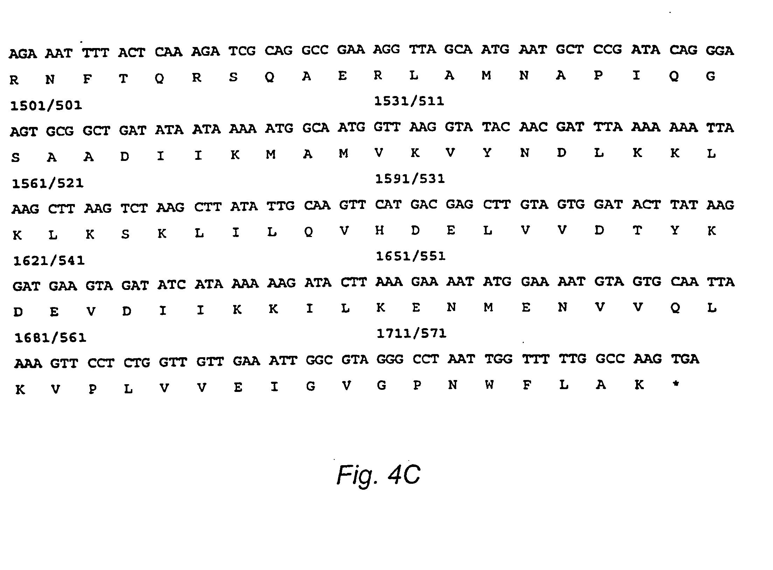

In a further aspect, the present invention provides a thermostable DNA polymerase which corresponds to the DNA polymerase from *Thermoanaerobacter thermohydrosulfuricus* in which up to one third of the amino acid sequence at the amino-terminus has been deleted. In particular, fragments of *Thermoanaerobacter thermohydrosulfuricus* having N-terminal deletions to give 578 amino acids (See Fig. 4A-C) (SEQ. ID. NO. 4) and 608 amino acids (See Fig. 3) (SEQ. ID. NO. 3) have been found to retain enzyme activity.

It is preferred that the 5'-3' exonuclease activity of the DNA polymerase is removed or reduced. This may be achieved by deleting the amino acid region of the enzyme responsible for this activity, e.g. by deleting up to one third of the amino acid sequence at the amino terminus, or by appropriate amino acid changes.

In addition to the N-terminal deletions and amino acid changes to remove the exonuclease activity, the enzyme may have conservative amino acid changes compared with the native enzyme which do not significantly influence thermostability or enzyme activity. Such changes include substitution of like charged amino acids for one another or amino acids with small side chains for other small side chains, e.g. ala for val. More drastic changes may be introduced at non-critical regions where little or no effect on polymerase activity is observed by such a change.

Joyce and Steitz, Annu. Rev. Biochem, 63:777-822, 1994, discuss various functions of DNA polymerases including the catalytic center, the binding site for the 3' terminus of the primer, and the dNTP binding site. In particular, it mentions mutations that affect the binding of dNTP in the ternary complex. European Patent Application 0655506A discloses that the presence of a polar, hydroxyl containing amino acid residue at a position near the binding site for the dNTP substrate is important for the polymerase being able to efficiently incorporate a dideoxynucleotide. Applicant has discovered that the modification of the dNTP binding site for the dNTP substrate in DNA polymerase obtainable from *Thermoanaerobacter thermohydrosulfuricus* by the inclusion of a polar, hydroxyl containing amino acid residue at a position near the binding site increases the efficiency of the polymerase to incorporate a dideoxynucleotide. Preferably the polar, hydroxyl containing amino acid is tyrosine. It has also been found that replacing the phenylalanine at the position corresponding to 706 of the native enzyme with tyrosine improves the incorporation of dideoxynucleotides when the enzyme is used for sequencing. In particular, a polymerase from *Thermoanaerobacter thermohydrosulfuricus* in which the exonuclease activity has been deleted e.g. by point mutation or deletion and which has the phenylalanine at the position corresponding to 706 of the native enzyme replaced by an amino acid which increases the efficiency of the enzyme to incbrpbrate dideoxynucleotides at least 20 fold compared to the wild type enzyme, e.g. tyrosine, is a particularly preferred enzyme for use in sequencing. Preferably, this modified enzyme has from between 540 and 582 amino acids, for example 560 to 582 amino acids and preferably 578 amino acids.

The DNA polymerases of the present invention can be constructed using standard techniques familiar to those who practice the art. By way of example, in order to prepare a polymerase with the phenylalanine to tyrosine mutation, mutagenic PCR primers can be designed to incorporate the desired Phe to Tyr amino acid change (FY mutation in one primer). Deletion of the exonuclease function is carried out by PCR to remove the amino terminus, or standard techniques of site directed mutagenesis to generate point mutations.

Improved expression of the DNA polymerases of the present invention can be achieved by introducing silent codon changes (i.e., the amino acid encoded is not changed). Such changes can be introduced by the use of mutagenic PCR primers. Silent codon changes such as the following increase protein production in *E. coli:*
substitution of the codon GAG for GAA;
substitution of the codon AGG, AGA, CGG or CGA for CGT or CGC;
substitution of the codon CTT, CTC, CTA, TTG or TTA for CTG;
substitution of the codon ATA for ATT or ATC;
substitution of the codon GGG or GGA for GGT or GGC.

The DNA polymerases of the present invention are suitably used in SDA, preferably in combination with a thermostable restriction enzyme. Accordingly, the present invention provides a composition which comprises a DNA polymerase of the present invention in combination with a thermostable restriction enzyme, for example *Bso*BI from *Bacillus stearothermophilus*. The invention also features a kit or solution for SDA comprising a DNA polymerase of the present invention in combination and a thermostable restriction enzyme.

The polymerases of the present invention are also useful in methods for generating and amplifying a nucleic acid fragment via a strand displacement amplification (SDA) mechanism. The method generally comprises:
a) specifically hybridizing a first primer 5' to a target nucleic acid sequence, the first primer containing a restriction enzyme recognition sequence 5' to a target binding region;
b) extending the 3' ends of the hybridized material using a DNA polymerase of the present invention, preferably one in which the exonuclease activity has been removed, in the presence of three dNTPs and one dNTP∝ S;
c) nicking at the hemiphosphorothioate recognition site with a restriction enzyme, preferably;
d) extending the 3' end at the nick using a DNA polymerase of the present invention, displacing the downstream complement of the target strand; and
e) repeating steps (c) and (d).

This SDA method proceeds at a linear amplification rate if one primer is used as above. However, if two primers are used which hybridize to each strand of a double-stranded DNA fragment, then the method proceeds exponentially (Walker, G.T., Little, M.C., Nadeau, J.G. and Shank D.D. (1992) *Proc. Natl. Acad. Sci. USA* 89:392-396).

The present invention also provides a method for determining the nucleotide base sequence of a DNA molecule. The method includes providing a DNA molecule, annealing with a primer molecule able to hybridize to the DNA molecule; and incubating the annealed molecules in a vessel containing at least one, and preferably four deoxynucleotide triphosphate, and a DNA polymerase of the present invention preferably one containing the phenylalanine to tyrosine mutation. Also provided is at least one DNA synthesis terminating agent which terminates DNA synthesis at a specific nucleotide base. The method further includes separating the DNA products of the incubating reaction according to size, whereby at least a part of the nucleotide base sequence of the DNA molecule can be determined.

In preferred embodiments, the sequencing is performed at a temperature between 40 and 75°C.

In other preferred embodiments, the DNA polymerase has less than 1000, 250, 100, 50, 10 or even 2 units of exonuclease activity per mg of polymerase (measured by standard procedure, see below) and is able to utilize primers having only 4, 6 or 10 bases; and the concentration of all four deoxynucleoside triphosphates at the start of the incubating step is sufficient to allow DNA synthesis to continue until terminated by the agent, e.g. a ddNTP.

Preferably, more than 2, 5, 10 or even 100 fold excess of a dNTP is provided to the corresponding ddNTP.

In a related aspect, the invention features a kit or solution for DNA sequencing including a DNA polymerase of the present invention and a reagent necessary for the sequencing such as dITP, deaza dGTP, a chain terminating agent such as a ddNTP, and optionally a pyrophosphatase.

The DNA polymerases of the present invention containing the phenylalanine to tyrosine mutation are suitably used in sequencing, preferably in combination with a pyrophosphatase. Accordingly, the present invention provides a composition which comprises a DNA polymerase of the present invention containing the phyenylalanine to tyrosine mutation in combination with a pyrophosphatase, preferably a thermostable pyrophosphatase from *Thermoplasma acidophilum*.

In another related aspect, the invention features a method for sequencing a strand of DNA essentially as described above with one or more (preferably 2, 3 or 4) deoxyribonucleoside triphosphates, a DNA polymerase of the present invention, and a first chain terminating agent. The DNA polymerase causes the primer to be elongated to form a first series of first DNA products differing in the length of the elongated primer, each first DNA product having a chain terminating agent at its elongated end, and the number of molecules of each first DNA products being approximately the same for substantially all DNA products differing in length by no more than 20 bases. The method also features providing a second chain terminating agent in the hybridized mixture at a concentration different from the first chain terminating agent, wherein the DNA polymerase causes production of a second series of second DNA products differing in length of the elongated primer, with each second DNA product having the second chain terminating agent at its elongated end. The number of molecules of each second DNA product is approximately the same for substantially all second DNA products differing in length from each other by from 1 to 20 bases, and is distinctly different from the number of molecules of all the first DNA products having a length differing by no more than 20 bases from that of said second DNA products.

In preferred embodiments, three or four such chain terminating agents can be used to make different products and the sequence reaction is provided with a magnesium ion, or even a manganese or iron ion (e.g. at a concentration between 0.05 and 100 mM, preferably between 1 and 10mM); and the DNA products are separated according to molecular weight in four or less lanes of a gel.

In another related aspect, the invention features a method for sequencing a nucleic acid by combining an oligonucleotide primer, a nucleic acid to be sequenced, between one and four deoxyribonucleoside triphosphates, a DNA polymerase of the present invention, and at least two chain terminating agents in different amounts, under conditions favoring extension of the oligonucleotide primer to form nucleic acid fragments complementary to the nucleic acid to be sequenced. The method further includes separating the nucleic acid fragments by size and determining the nucleic acid sequence. The agents are differentiated from each other by intensity of a label in the primer extension products.

In a further aspect, the present invention provides a method for preparing complementary DNA by combining an oligonucleotide primer, a sample of RNA, a DNA polymerase of the present invention, and between one and four deoxyribonucleoside phosphates, under conditions favoring preparation of the complementary DNA.

The DNA polymerases of the present invention which act as reverse transcriptases lack appreciable, and preferably have no RNaseH activity and, as such, are useful in RT/PCR, the generation of hybridization probes and RNA sequencing. In a yet further aspect, the present invention provides a purified thermostable reverse transcriptase having a reverse transcriptase activity of greater than 1000 units per milligram. Preferably, the reverse transcriptase lacks RNaseH activity; the reverse transcriptase is from *Thermoanaerobacter thermohydrosulfuricus;* the reverse transcriptase has an N-terminal deletion or amino acid changes that remove the exonuclease function. In a further aspect, the invention features a method for reverse transcription/polymerase chain reaction (RT/PCR) which utilizes a DNA polymerase of the present invention and a DNA polymerase suitable for PCR in the same reaction vessel. Preferably, the DNA polymerase of the present invention is from *Thermoanaerobacter thermohydrosulfuricus,* the polymerase has one or more amino acids deleted from the amino terminus or amino acid changes to remove the exonuclease activity, the DNA polymerase suitable for PCR is Taq DNA polymerase. In another aspect, the present invention features a kit or solution for RT/PCR comprising a DNA polymerase of the present invention and a DNA polymerase suitable for PCR. Preferably, the DNA polymerase of the present invention is from *Thermoanaerobacter thermohydrosulfuricus,* the polymerase has one or more amino acids deleted from the amino terminus or amino acid changes to remove the exonuclease function, and the DNA polymerase suitable for PCR is Taq DNA polymerase.

In a further preferred embodiment the invention features polymerases that have exonuclease activity removed by replacing an aspartic acid residue at position 8 with an alanine residue. This is a preferred mutation for it not only removes the exonuclease activity but removes an RNaseH activity, which may effect reverse transcription.

In another aspect, the invention features a method for polymerase chain reaction in the presence of a polymerase stabilizing agent, utilizing an enzymatically active DNA polymerase having at least 80% identity in its amino acid sequence to the DNA polymerase of *Themoanaerobacter thermohydrosulfuricus* and an exonuclease activity removed. The polymerase stabilizing agents include, but are not limited to, glycerol (10 to 50% final concentration), trimethylamine-N-oxide (TMANO; up to 4 M final concentration), and N-methylmorpholine-N-oxide (MMO; up to 3 M final concentration). Preferably, glycerol is used at a final concentration of 30%. By polymerase stabilizing agent is meant an agent which allows the use of the polymerase in PCR and RT/PCR. These agents reduce the denaturing temperature of the template and stabilize the polymerase. By stabilize is meant make temperature stable. By final concentration is meant the final concentration of the agent in the PCR or RT/PCR solution.

The invention also features kits, with polymerase stabilizing agents, for polymerase chain reaction having an enzymatically active DNA polymerase with at least 80% identity in its amino acid sequence to the DNA polymerase of *Themoanaerobacter thermohydrosulfuricus* and an exonuclease activity removed. The polymerase stabilizing agents include, but are not limited to, glycerol (10 to 50% final concentration),TMANO (up to 4 M final concentration), and MMO (up to 3 M final concentration). Preferably, glycerol is used at a final concentration of 30%. Also encompassed are solutions for use in polymerase chain reaction, having polymerase stabilizing agents including, but not limited to, glycerol (10 to 50% final concentration), TMANO (up to 4 M final concentration), and MMO (up to 3 M final concentration), and an enzymatically active DNA polymerase having at least 80% identity in its amino acid sequence to the DNA polymerase of *Themoanaerobacter thermohydrosulfuricus* and an exonuclease activity removed. Preferably, glycerol is used at a final concentration of 30%.

In preferred embodiments the exonuclease activity is removed by an N-terminal deletion to give between 540 and 582 amino acids, by deleting up to one third of the amino acid sequence at the N-terminal, by substitutition of an amino acid in the amino terminal one third of the protein, or by substitution of the aspartic acid residue at position 8 with an alanine residue, and the glycerol concentration is 30%.

Applicants have discovered that the DNA polymerases of the present invention can be used to carry out polymerase chain reaction (PCR) when the reaction is carried out in the presence of a polymerase stabilizing agent, such as glycerol, TMANO, or MMO, and the like. Preferably, the glycerol concentration is in the range of 10 to 50%, and most preferably it is at a final concentration of 30%. TMANO and MMO can be used at concentrations up to 4 M and 3 M final concentration, respectively. These agents have been shown to stabilize the polymerase from *Themoanaerobacter thermohydrosulfuricus* (herein and U.S. Patent Application No. 08/766,014, filed December 13, 1996, *supra*). Thus, the polymerases of the present invention are also suitable for reverse transcription/polymerase chain reaction (RT/PCR) under these conditions.

Thus, in another aspect the invention features a method for reverse transcription/polymerase chain reaction, in the presence of a polymerase stabilizing agent, utilizing an enzymatically active DNA polymerase having at least 80% identity in its amino acid sequence to the DNA polymerase of *Themoanaerobacter thermohydrosulfuricus* and an exonuclease activity removed. In preferred embodiments, the polymerase stabilizing agents include, but are not limited to, glycerol (10 to 50% final concentration), TMANO (up to 4 M final concentration), and MMO (up to 3 M final concentration). Preferably, glycerol is used at a final concentration of 30%.

The invention also features kits for reverse transcription/polymerase chain reaction, with a polymerase stabilizing agent, having an enzymatically active DNA polymerase with at least 80% identity in its amino acid sequence to the DNA polymerase of *Themoanaerobacter thermohydrosulfuricus* and an exonuclease activity removed. Also encompassed are solutions for use in reverse transcription/polymerase chain reaction comprising a polymerase stabilizing agent and an enzymatically active DNA polymerase having at least 80% identity in its amino acid sequence to the DNA polymerase of *Themoanaerobacter thermohydrosulfuricus* and an exonuclease activity removed. In preferred embodiments, the polymerase stabilizing agents include, but are not limited to, glycerol (10 to 50% final concentration), TMANO (up to 4 M final concentration), and MMO (up to 3 M final concentration). Preferably, glycerol is used at a final concentration of 30%.

In preferred embodiments the exonuclease activity is removed by having an N-terminal deletion to give between 540 to 582 amino acids, by deleting up to one third of the amino acid sequence at the N-terminal, by substitutition of an amino acid in the amino terminal one third of the protein, by substitition of the aspartic acid residue at position 8 with an alanine residue, and the glycerol concentration is 30%.

Other features and advantages of the invention will be apparent from the following description of the preferred embodiments, and from the claims.

### Description of the Preferred Embodiments

The drawings will first be briefly described.

### Drawings

Fig. 1A-E is the DNA sequence for 5300 bp of genomic DNA flanking and encoding the full length DNA polymerase from *Thermoanaerobacter thermohydrosulfuricus* (SEQ. ID. NO. 1).
Fig. 2A-D is a contiguous open reading frame capable of encoding the full length polymerase from *Thermoanaerobacter thermohydrosulfuricus* (SEQ. ID. NO. 2). Translation is of an open reading frame spanning nucleotides 1056-3674 of genomic DNA in Fig. 1A-E, encoding native polymerase.
Fig. 3 is the contiguous open reading frame of nucleotides 796-2616, amino acids 266-872 plus an initiator methionine at the amino terminus, the exo⁻ mutant of the enzyme containing 608 amino acids (SEQ. ID. NO. 3).
Fig. 4A-C is the contiguous open reading frame of the F412Y mutation of the exo-mutant polymerase, containing 578 amino acids with. (SEQ. ID. NO. 4). At amino acid 412 there is a tyrosine replacing phenylalanine.
Fig. 5 is a schematic of cloned polymerases. Letters A-D represent start sites of the various constructs shown in the table in Example 2.
Fig. 6 is a graphical representation comparing relative RT activity of the exo- mutant enzyme and other enzymes that have reverse transcription activity. The x-axis indicates the polymerase (MMLV, AMV, Tth, USB). The y-axis represents reverse transcriptase activity in cpm.
Fig. 7A-C is the DNA sequence encoding a full length native polymerase from *Thermoanaerobacter thermohydrosulfuricus* (SEQ. ID. NO. 23).

### Examples

The following examples serve to illustrate the DNA polymerases of the present invention and are not intended to be limiting.

### Example 1: General Methods Used During Purification and Characterization

### Assay of DNA Polymerase Activity

DNA polymerase activity was assayed by measuring the amount of incorporation of a radiolabelled deoxynucleotide into acid percipitable material using activated salmon sperm DNA as a template (Richardson, C. C. (1966) DNA polymerase from *Escherichia coli,* pp. 263-276 *In* G. L. Cantoni and D. R. Davies (ed.), Procedures in nucleic acid research. Harper and Row, New York). A unit of DNA polymerase is the amount of enzyme that catalyzes incorporation of 10 nmoles of deoxynucleotide triphosphate into acid-precipitable material in 30 min at 70 °C. An assay consists of 2-10 µL of protein solution containing DNA polymerase being incubated with 50 µL of assay mix (20 mM Tris HCl pH 8.5 @ room temperature, 200 µM each deoxynucleotide triphosphate, 10 mM KCl, 5 mM MgCl₂, 0.2 mg/mL activated salmon sperm DNA, 1 µCi 3000 Ci/mmole [α-³³P]dATP) at 70 °C for 10 min. The reaction is stopped by adding the contents of the assay to a tube containing 1 mL each of carrier (50 µg/mL fish sperm DNA, 2 mM EDTA) and precipitant (20% (w/v) trichloroacetic acid, 2% (w/v) sodium pyrophosphate). After incubation on ice for at least 5 min, the solution is filtered through a glass fiber filter (e.g. GF/B, Whatman), washed with several mL ice-cold wash solution (1 N hydrochloric acid, 100 mM sodium pyrophosphate), placed in a counting vial with aqueous liquid scintillation cocktail (e.g. Biosafe II, Research Products International Corp.) and counted in a liquid scintillation counter. DNA polymerase activity of the test solution is calculated from the measured specific activity of the assay mix. Only activity values measured to be within the linear range of 0.01 to 0.2 units per assay reaction were accepted as significant.

### Measurement of Protein Concentration

Solution protein concentrations are measured spectrophotometrically by determining the absorbance of the test solution at a wavelength of 205 nm (A₂₀₅) (Scopes, R. K. (1994) pp. 46-48 Protein Purification. Springer-Verlag, New York). The extinction coefficient of polypeptides is taken as _{E205(} 1 mg/mL) = 31.

### Assay of Exonuclease Activity

Exonuclease activity was measured as described (Kong, H., Kucera, R. B. and Jack, W. E. (1993) *J. Biol. Chem.* 268, 1965-1975) using, as substrate, *Hind*III digested phage λ DNA labeled with ³H by the action of CpG methylase or a 500 bp PCR product generated from pBR322 labeled with ³H by incorporation of tritiated TTP. Exonuclease assays were performed in the same buffer (20 mM Tris·HCl pH 8.5 @ room temperature, 10 mM KCl, 5 mM MgCl₂) and at the same temperature (70 °C) as polymerase assays.

### Assay for Directionality of Exonuclease

The directionality of the associated exonuclease activity was determined by monitoring the time-dependent release of radioactivity from a DNA substrate differentially labeled on the 5' and 3' ends as described (Kong et al., supra).

### Assay for Strand Displacement Activity

An extension assay has been developed to evaluate polymerases for their utility in the SDA (strand displacement amplification) reaction (Becton-Dickinson). This extension reaction tests the ability of the polymerase to displace a downstream strand and its ability to initiate extension at a nick site. This displacement and initiation at a nick is staged by annealing two primers labeled at their 5' ends immediately adjacent to each other. If the polymerase has strand displacement activity and is able to initiate at a nick, both primers are extended and two extension products can be visualized. If the polymerase cannot initiate at a nick (that is, it has a preference for initiation at a gap) or it lacks displacement activity then only the downstream primer is extended and only one product is detected.

Using the plasmid pBR322 as the target, two primers pBR1 and pBR2 were synthesized that anneal immediately adjacent to each other on the plasmid. Primer pBR1 is the upstream primer and corresponds to bases 3661 to 3690 in pBR322 (GGTTCCCAACGATCAAGGCGAGTTACATGA) (SEQ. ID. NO. 5). Primer pBR2 anneals downstream and corresponds to pBR322 3691-3720 (TCCCCCATGTTG TGCAAAAAAGCGGTTAGC) (SEQ. ID. NO. 6). These 30 mer oligos were purified by electroelution from a denaturing polyacrylamide gel. The primers are labeled in a kinase reaction using 10 µM primer, 50 mM Tris·HCl pH 7.5, 10 mM MgCl₂, 70 µCi [γ-³²P] ATP and 10 units T4 polynucleotide kinase for 30 min at 37 °C. A final concentration of 0.2 µM (each) of these two labeled primers are combined with 200 ng of *Pst*I/*Hinc*II digested pBR322. These are denatured at 98 °C for 3 min in a buffer containing 25 mM potassium phosphate, pH 7.4, 2-8 mM MgCl₂, 0.2 to 1 mM dNTPs and cooled to the reaction temperature (50-70 °C) for 2 min. One unit of the polymerase is added and the reaction is continued for 10 min. The reactions are stopped by addition of an equal volume of 95% formamide, 50 mM EDTA, bromophenol blue dye. Twenty-five µL are electrophoresed over a 6% denaturing gel and the gel is exposed to autoradiographic film.

### Example 2: Purification of Native Enzyme

### Crude Purification

### Growth of Thermoanaerobacter thermohydrosulfuricus Cells

A lyophilized culture of *Thermoanaerobacter thermohydrosulfuricus* strain JW102 was purchased from the Deutsche Sammlung von Mikroorganismen und Zellkulturen, GmbH (DSM). The growth medium used was Clostridium Thermohydrosulfuricum Medium (medium #61 in the DSM catalog of strains, 1993 edition) which consists of (per L): 10 g tryptone, 10 g sucrose, 2 g yeast extract, 0.2 g FeSO₄7H₂O, 0.2 g Na₂ SO₃, 0.08 g Na₂S₂O₃·5H₂O and 1 mg Resazurin. The culture was grown at 70 °C under anaerobic conditions. After growth, the media was cooled to approximately room temperature and the culture harvested by continuous flow centrifugation. Cell paste was stored at -80 °C prior to subsequent procedures.

### Preparation of Extract

Frozen cell paste (5 grams) was combined with 100 mL of buffer A (50 mM Tris·HCl pH 8.0, 10 mM NaCl, 1 mM DTT, 1 mM EDTA, 10% (w/v) glycerol). The paste was stirred at room temperature until resuspension was complete. The resulting cell suspension was cooled to 0 °C by incubation in an ice bath and subjected to approximately 5 min of sonication to lyse the cells. Cell debris was removed by centrifugation at 70,000 x g for 20 min at 4 °C. The supernatant cleared lysate was designated fraction 1.

### Liquid Chromatography Purification

Fraction 1 was loaded onto a low-pressure liquid chromatography column (70 mL bed volume, 26 mm ∅) of DEAE cellulose (DE-52, Whatman) equilibrated with buffer A. The column was washed with several column volumes of buffer A and the DNA polymerase eluted with a linear salt gradient from 10 mM to 1000 mM NaCl in buffer A at a flow rate of 0.9 mL/min. This and all subsequent chromatography steps were carried out at ambient room temperature (approximately 25 °C). The peak of activity eluted at approximately 32 mM NaCl. Active fractions were pooled and designated fraction 2.

Fraction 2 was loaded directly onto a column (22 mL bed volume, 16 mm Ø) of heparin sepharose (Heparin Sepharose CL-6B, Pharmacia) equilibrated with buffer A. The column was washed with several column volumes of buffer A and the DNA polymerase activity eluted with a linear salt gradient from 10 mM to 700 mM NaCl in buffer A at a flow rate of 0.4 mL/min. The peak of activity eluted at approximately 400 mM NaCl. Active fractions were pooled and designated fraction 3.

Fraction 3 was loaded directly onto a column (2 mL bed volume, 16 mm Ø) of hydroxylapatite (BioGel HA, Bio-Rad) equilibrated with buffer B (50 mM potassium phosphate, pH 7.4, 1 mM DTT, 10% (w/v) glycerol). The column was washed with several column volumes of buffer B and the DNA polymerase eluted with a linear gradient from 50 mM to 700 mM potassium phosphate in buffer A at a flow rate of 0.4 mL/min. The DNA polymerase containing fractions were pooled and concentrated by ultrafiltration (Centricon-50, Amicon) to a volume of 100 mL to which 100 mL of 100% glycerol was added to obtain the final product. This final preparation was determined to contain about 1000 units of DNA polymerase which was judged to be approximately 80% pure on a Coomassie stained denaturing polyacrylamide gel (SDS-PAGE).

### Optimized Purification

### Preparation of Extract

Frozen cell paste (45 grams) was resuspended in 250 mL of lysis buffer (50 mM Tris-HCl, pH 8, 10 mM NaCl, 5 mM EDTA, 0.2% (v/v) NP40, 1 mM DTT). Lysozyme was added to 200 mg/mL final concentration and the solution was stirred at room temperature for 60 min. DNase I was added to 10 mg/mL final concentration and incubation was continued for 10 min. Insoluble material was removed by ultracentrifugation at 70,000 x g for 40 min. The decanted supernatant is designated fraction I.

### Polyethyleneimine Precipitation

A pilot polyethyleneimine (PEI) precipitation experiment was done to determine the final PEI concentration for this lysate to precipitate all polymerase activity with the least coprecipitating protein content. PEI was added from a 10% (v/v) stock to Fraction I to 0.4% final concentration. The solution was stirred for 20 min at 4 °C. The PEI precipitate was collected by centrifugation at 8,000 x g for 20 min. The polymerase activity was extracted from the pellet with 150 mL of 300 mM NaCl in buffer A (50 mM Tris-HCl, pH 8, 1 mM DTT, 1 mM EDTA and 10% glycerol). This solution was centrifuged again at 17,000 x g. Ammonium sulfate was added to the supernatant to 80% saturation and the solution was stirred for one hour at 4 °C to precipitate proteins from residual PEI. The ammonium sulfate fraction was centrifuged at 48,000 x g for 30 min and the pellet resuspended in 400 mL 10 mM NaCl, 50 mM Tri•HCl, pH 8. This is designated Fraction II.

### Liquid Chromatography Purification

A column (150 mL bed volume) of Heparin-Seharose CL-6B (Pharmacia) was equilibrated with 150 mM NaCl in buffer A. Fraction II was loaded at 150 mL/h and washed with two bed volumes of buffer A. The polymerase activity was then eluted with a linear gradient of 150 mM to 700 mM NaCl in buffer A yielding a polymerase peak at about 400 mM NaCl. The peak fractions were pooled and diluted fivefold with 100 mM NaCl in buffer B (50 mM Tris-HCl, pH 7.5, 0.1 mM DTT, 1 mM EDTA and 5% glycerol). This pool is designated Fraction III.

A column (4 mL bed volume) of ssDNA agarose (Pharmacia) was equilibrated with 100 mM NaCl in buffer B. One-third of the Fraction III was loaded at a flow rate of 12 mL/hr. The column was washed with the same buffer and polymerase activity eluted with a linear gradient of 100 mM to 1000 mM NaCl in buffer B. The polymerase activity peak eluted at approximately 375 mM NaCl. The peak fractions were pooled and concentrated with Centricon-50 (Amicon) and stored by adding an equal volume of 100% glycerol.

### Preparation of exo-Enzyme by Subtilisin Cleavage

Approximately 850 units of purified native *Thermoanaerobacter thermohydrosulfuricus* DNA polymerase I was exchanged into 150 mM potassium phosphate pH 6.5 by three rounds of dilution in this buffer and concentration by ultrafiltration yielding a solution containing about 250 mg of protein in 550 µL. Subtilisin (10 µL of 0.06 mg/mL solution) was added to the polymerase and the mixture was incubated at 37 °C for 1 hour. After incubation, the reaction was diluted to 10 mL final volume with buffer A and loaded onto a column of heparin sepharose (4 mL bed volume, 16 mm Ø) equilibrated in buffer A containing 150 mM NaCl. The column was washed with several column volumes of this buffer and the polymerase activity eluted with a linear gradient of 150 mM to 700 mM NaCl in buffer A. Active fractions were pooled.

### Example 3: Characterization of Native Enzyme

### General properties

The native enzyme migrates as an approximately 90,000 Da polypeptide (just below phosphorylase B) on SDS-PAGE. The enzyme possesses strand displacement activity. It possesses a 5'-3' exonuclease activity at approximately 1/80 the level of polymerase activity (unit:unit). The temperature optimum of DNA synthesis is near 75 °C under assay conditions. The optimum Mg²⁺ concentration of DNA synthesis is 1 mM. The optimum Mn²⁺ concentration of DNA synthesis is 0.5 mM.

### Assay for thermal stability

To assess the thermal stability of the enzyme, a sample of native *Thermoanaerobacter thermohydrosulfuricus* DNA polymerase was incubated at elevated temperatures in several buffers. The buffers all consisted of 25 mM Tris-HCl, pH 8.5, 50 mM KCl, 5 mM MgCl₂, 1 mM 2-mercaptoethanol, 0.5% (w/v) Nonidet P-40 and 100 mg/mL BSA with the following additions:
- BSA buffer :: no additions.
- GLY buffer :: add glycerol to 40% (v/v)
- MMO buffer :: add N-methylmorpholine-N-oxide (MMO) to 3 M
- TMANO buffer :: add Trimethylamine-N-oxide (TMANO) to 4 M

Native enzyme (0.02 units/µl) was incubated in these buffers at various temperatures. At 5, 10 and 15 min, 5 µL aliquots were withdrawn and placed into a fresh tube on ice. When all samples had been acquired, the heated aliquots were assayed in the usual manner. Results are expressed as the fraction activity remaining at a given time point at a given temperature.

The enzyme was least stable in BSA buffer, retaining half the initial measured polymerase activity after about 5 min at 80 °C. In GLY buffer, the enzyme retained half the initial activity after about 5 min at 85 °C. In MMO and TMANO buffers, incubation at 85 to 90 °C was required to eliminate half the initial measured activity in 5 min. Incubation in any of the these buffers at 75 °C for 15 min resulted in not more than 20% loss of initial DNA polymerase activity.

### Example 4: Characterization of Subtilisin-Cleaved Enzyme

### General properties

The cleaved enzyme migrates as a doublet consisting of polypeptides migrating at approximately 64 kDa and 55 kDa on SDS-PAGE. It is free of detectable exonuclease activity. The enzyme possesses strand displacement activity. The enzyme has similar thermal stability to the native enzyme.

### Example 5: Preparation of Cloned 64 kDa Enzyme

### Preparation of genomic DNA

Frozen cell paste of *Thermoanaerobacter thermohydrosulfuricus* (approximately 350 mg) was resuspended in 700 µL of lysis buffer (50 mM Tris·HCl pH 8.0, 50 mM EDTA, 3% SDS (w/v), 1% 2-mercaptoethanol (v/v)) and incubated for 1 hour at 65 °C. This solution was extracted three times with 25:24:1 phenol:chloroform:isoamyl alcohol, twice with chloroform and precipitated with two volumes of 100% ethanol. The pellet was dried briefly *in vacuo* and dissolved in 700 µL TE (10 mM Tris-HCl, pH 8.0, 1 mM EDTA). The concentration of genomic DNA was determined spectrophotometrically by measuring the absorbance at 260 nm (1A₂₆₀= 50 µg/mL) and by comparison of UV fluorescence of bands on an ethidium bromide stained agarose gel relative to standard concentration markers.

### Preparation of DNA polymerase I specific probe

Genomic DNA was used as a substrate for a PCR reaction using degenerate oligonucleotides designed against conserved regions of DNA polymerase I family polymerases (Uemori, T., Ishino, Y., Fujita, K., Asada, K. and Kato, I. (1993) *J. Biochem.* 113, 401-410). A standard Taq PCR reaction (20 µL) contained about 1 ng genomic DNA and 1mM of each primer EPOLF (GACCC(ATC)AAC(CT)T(CG)CA(AG)A A(CT)AT(ATC)CC (SEQ.ID.NO. 7) and EPOLR ((GT)A(CG)(CG)A(GT) (CT)TC (AG) TCGTG (GATC) AC (CT) TG) (SEQ. ID. NO. 8). After one round of PCR, the product was resolved on an agarose gel. The product around the expected 600 bp region was excised from the gel, crushed, soaked in TE and used as substrate for a second round of PCR using the same conditions. The resulting ca. 600 bp product was directly sequenced using the EPOLF and EPOLR primers (Sequenase PCR Product Sequencing Kit, Amersham). One open reading frame was found to be homologous to the targeted region of DNA polymerases I of known sequence as judged by BLAST analysis.

### Preparation of subgenomic libraries and screening

This PCR product was labeled using a radioactive or non-radioactive hybridization kit (Gene Images, USB) and used to probe blots of agarose gels of genomic DNA digested with various restriction enzymes. Digestion of genomic DNA with *Hind*III produced a unique hybridizing fragment of approximately 1.7 kb. DNA fragments of approximately 1.7 kb were excised from an agarose gel and cloned into pBluescript II KS (+) (Stratagene). Individual colonies that hybridized to the probe were purified and sequenced. Similarly, double digestion of *Thermoanaerobacter thermohydrosulfuricus* genomic DNA with *Eco*RI and *Xho*I produced a unique hybridizing fragment of approximately 1.9 kb and digestion with *Eco*RI produced a unique hybridizing fragment of approximately 3 kb. The sequences of these clones were merged and formed a coherent open reading frame which had high homology to the C-terminal portion DNA polymerases I of known sequence as judged by BLAST analysis. The clone containing the 1.7 kb *Hind*III fragment was digested with *Eco*RI and *Xho*I to produce a 1.4 kb fragment which was gel purified, ³²P labeled by nick-translation and used to probe a blot of *Thermoanaerobacter thermohydrosulfuricus* genomic DNA digested with *Pst*I and *Xho*I. This identified a unique hybridizing fragment of 3 kb that contained the remaining N-terminal portion of the gene. The nucleotide sequence is presented in Fig. 1A-E (SEQ. ID. NO. 1) and a contiguous open reading frame potentially encoding the native polymerase is presented in Fig. 2A-D (SEQ. ID. NO. 2).

### Preparation of expression vectors

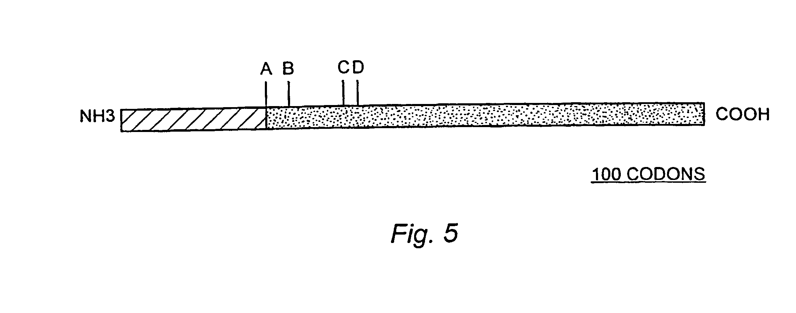

Several PCR products were made from the genomic DNA that allowed cloning portions of polymerase coding sequence not including 5'-3' exonuclease domain (See Fig. 5). Letters A-D represent start sites of the various constructs outlined in the table below. These truncated polymerase coding sequences were placed under the control of the λ P_{L} promoter of the expression vector pRE2 (Reddy, P., Peterkofsky, A. and McKenney, K. (1989) *Nucleic Acids Res.* 17, 10473- 10488). These vectors were propagated in *E. coli* strain DH-1 *λ*+ (*λ cI* +).

To minimize errors introduced by Taq DNA polymerase in PCR, a modified long PCR was carried out. The PCR reaction contained: 20 mM Tricine (pH 8.8), 85 mM KOAc, 200 mM each dNTP, 5% DMSO, 0.5 mM each primer, 1.5 mM MgOAc (added as Hot-Start), 2.5 units Hot Tub (or rTth) DNA polymerase per 100 mL reaction (Amersham), 0.025 U Deep Vent DNA (New England Biolabs) polymerase per 100 µL reaction, 20-100 ng *Thermoanaerobacter thermohydrosulfuricus* genomic DNA per 100 µL reaction. The cycling parameters consisted of: 94 °C 30s, then 68 °C 10 m 40 s x 8 cycles; 94 °C 30 s, then 68 °C 12m 00 s x 8 cycles; 94 °C 30s, then 68 °C 13 m 20 s x 8 cycles; 94 °C 30 s, then 68 °C 14 m 40 s x 8 cycles.

All PCR products were generated with the reverse primer DDREV(GGGGTACCT CTAGATCACTTGGCCAAAAACCA) (SEQ. ID. NO. 9) at the 3' end and various primers utilized at the 5' end:

DDMET (GGAATTCCATATGGCTTACCTGCTGAACCCGTCTGAGTCTAAC) (SEQ.
ID. NO. 10);

DD55kd (GGAATTCCATATGGCTCTTTCTTATAAAGGAATAGAG) (SEQ. ID. NO.
11);

DD64kd (GGAATTCCATATGAAAGTTGAAAAATGGTCA) (SEQ. ID. NO. 12);

DD64pref(GGAATTCCATATGAAAGTTGAAAAATGGTCTAAAGTAGACATCAAAGAATT
AG)(SEQ.ID.NO.13);

DDHF (GGAATTCCATATGAAGCTTTTAGAGCTTTT)(SEQ. ID. NO. 14);

DD67kd (GGAATTCCATATGAAACTTTTAGAGCTTTTCAACAAATTAGAATTC)
(SEQ. ID. NO. 15).

The resultant PCR products were digested with *Nde*I and *Kpn*I and cloned into pRE2 digested with the same restriction enzymes.

Silent changes (see Table below) denotes that the 5' primer encodes some codons which are synonymous to the native sequences, but are more frequently utilized by *E. coli*. By this method, six different clones were constructed which encode four different polypeptides.

| Site | Plasmid | MW | codons | 5' Primer | Note |
|---|---|---|---|---|---|
| D | pMET11 | 55 kDa | 483 | DDMET | Silent changes, native MET |
| C | pRED5504 | 58 kDa | 502 | DD55kd | Native sequence + MET |
| B | pRED6404 | 64 kDa | 579 | DD64kd | Native sequence + MET |
| B | pPREF2 | 64 kDa | 579 | DD64pref | Silent changes + MET |
| A | pRED4 | 67 kDa | 609 | DDHF | Native sequence + MET |
| A | pRED6706 | 67 kDa | 609 | DD67kd | Silent changes + MET |

### Expression of Cloned 64 kDa Enzyme

*E. coli* strain MZ-1 (cI857 lysogen) harboring the plasmid pRED6404 was cultured in LB medium (per L: 10 g tryptone, 5 g yeast extract, 10 g NaCl) supplemented with 50 µg/mL ampicillin. The culture was grown at 30-32 °C until the OD₆₀₀ reached 1.5 at which time the culture temperature was raised to 40-42 °C. Growth continued at this temperature for 2 hours before the culture was harvested in a continuous flow centrifuge. Cell paste was stored at -80 °C prior to subsequent procedures.

### Example 6: Preparation of Cloned Full-length Enzyme

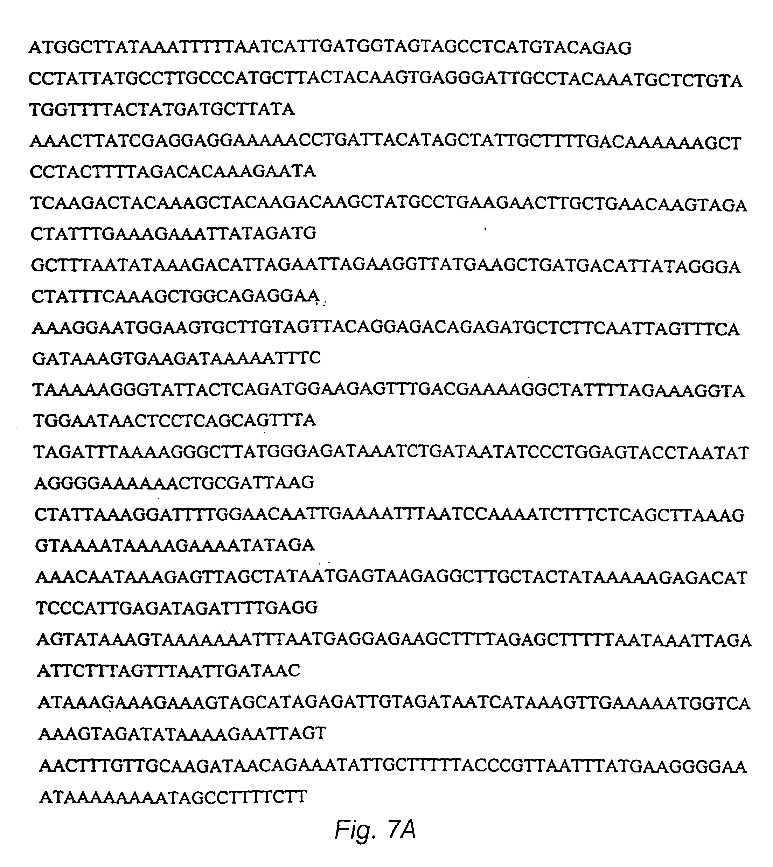

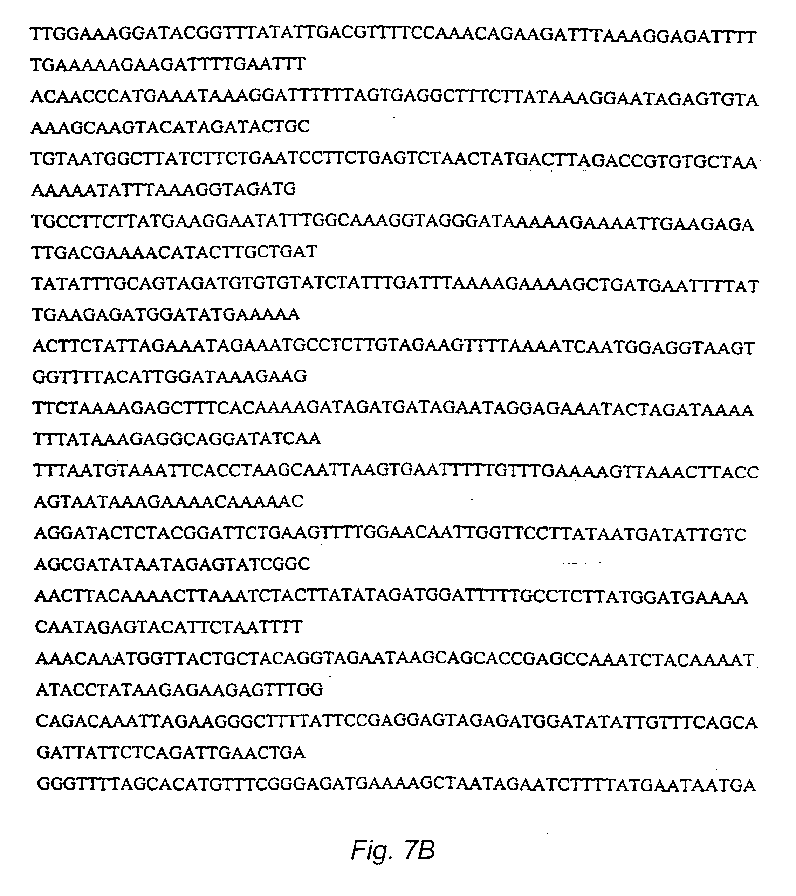

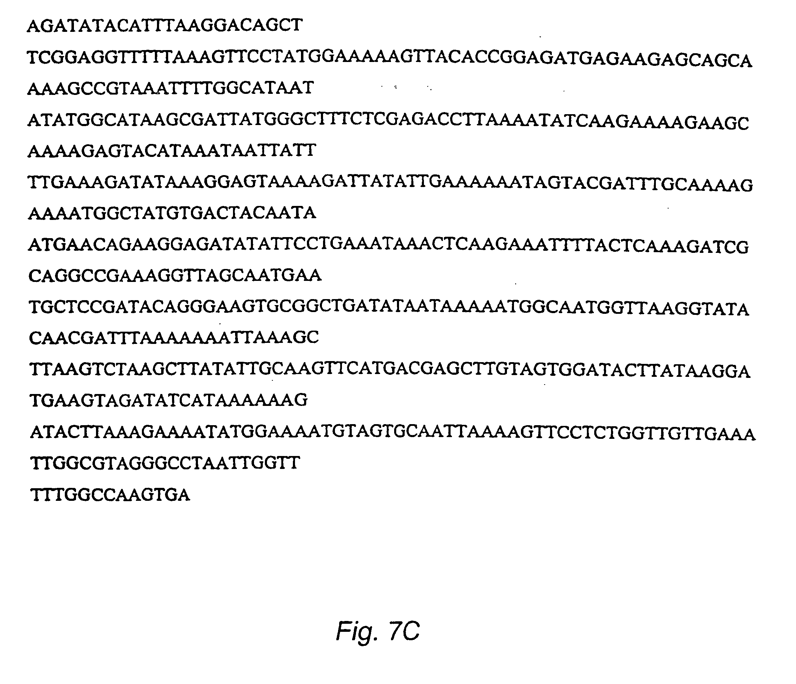

Full length enzyme was cloned by PCR from the clone of genomic DNA (SEQ. ID. NO. 1) (Fig. 1A-E). A 5' primer DDF-FOR (GGAATTCCATATGGCTTATAAATTTTTAATCATTGATGGTAGTAGC) (SEQ. ID.NO. 24) was synthesized. This primer encodes an *Nde* I site, inserts an alanine codon after the initiating methionine and changes two codons to synonymous codons favored by *E. coli* (ATA -> ATC, Ile at aa position 6 and GGA -> GGT, Gly at aa position 9). This primer was used in conjunction with a vector primer from the assembled clone of genomic DNA to produce a PCR product which was subsequently digested with *Nde* I and *Xho* I and sublconed into similarly digested pRED6404 to form pLS-3. The DNA for the full length enzyme is shown in Fig. 7 A-C (SEQ. ID. NO. 23).

### Example 7: Purification of Cloned 64 kDa Enzyme

### Preparation of Extract

Frozen cell paste (370 grams) was combined with 1800 mL of buffer A (50 mM Tris-HCl, pH 8.0, 10 mM NaCl, 1 mM DTT, 1 mM EDTA, 10% (w/v) glycerol). The paste was stirred at room temperature until resuspension was complete. The cells were lysed using a French pressure cell. Cell debris was removed by centrifugation at 70,000 x g for 20 min at 4 °C.

### Liquid chromatography purification

The cleared lysate was passed through a column of DEAE cellulose at 300 mM salt. The collected flow through was heated to 70 °C for 10 min, cleared again by centrifugation and loaded onto a column heparin sepharose. The DNA polymerase was eluted from this column with a salt gradient as before and was found in most cases to be pure. If not, column chromatography on hydroxylapatite was performed.

### Example 8: Characterization of Cloned 64 kDa Enzyme

### General properties

Cloned enzyme refers to the purified gene product of pRED6404. The cloned enzyme migrates as an approximately 64,000 Da polypeptide (just below bovine serum albumin, MW 67,000) on SDS-PAGE. The specific activity of the cloned enzyme is 28,000 units/mg. It is free of detectable exonuclease activity. The temperature optimum of DNA synthesis is near 72 °C under assay conditions. The optimum Mg²⁺ concentration of DNA synthesis is 2 mM. The optimum Mn²⁺ concentration of DNA synthesis is 0.7 mM.

### Reverse transcriptase activity

The cloned enzyme functioned as a reverse transcriptase in an assay containing poly(rA):oligo(dT₁₂₋₁₈) and [³H]TTP at 42 °C. This enzyme has RT activity in the presence of Mg²⁺ with or without the addition of Mn²⁺.

### Measurement of reverse transcriptase activities of different enzymes using an SPA format

Amersham reverse transcriptase scintillation proximity assay (SPA) kit (NK9020), and MMLV (E 70456), and AMV (E 70041) enzymes were supplied by Amersham International. The cloned 64 kD enzyme prepared above was designated as USB enzyme. Tth (N808-0098) enzyme was obtained from Perkin-Elmer.

MMLV and AMV enzymes were tested at 37 °C and 42 °C respectively in the presence of magnesium. The Tth and USB enzymes were both used at 60 °C with manganese present as the co-factor. The assay buffer used was the same as supplied in the SPA kit except the appropriate co-factor was substituted in the buffer mix. Essentially, the assay was set up as follows in duplicate using the protocol provided in the SPA kit in sarstedt tubes in a total reaction volume of 100 µL.
80 µL of 0.77 µM [³H]TTP in a buffer consisting of:
50 mM Tris·HCl pH 8
80 mM KCl
10 mM MgCl₂ or 4 mM MnCl₂
10 mM DTT
0.05% w/v NP40
10 µL of 0.2 µM primer/template (biotinylated (dT₁₆) oligonucleotide annealed to poly r(A)) in
10 mM Tris·HCl pH 8
1 mM MgCl₂

10 µL of 1 unit (as defined by the manufacturer) of enzyme diluted in 50 mM Tris-HCl, pH 8, 10 mM DTT was added to each tube to start the reaction and incubated at the indicated temperature for 30 min after which the reaction was terminated with the addition of 10 µL 0.56 M EDTA, pH 8. 10 µL of the supplied streptavidin SPA beads were then added to each tube, and after incubating for a further 10 min at room temperature, were counted in a LKB 1205 scintillation counter.

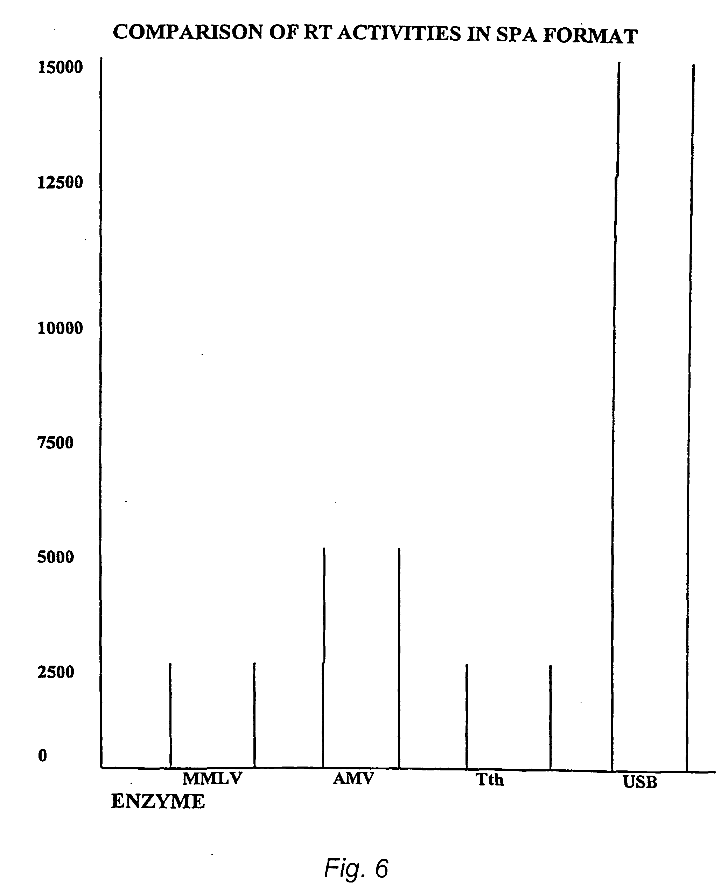

Appropriate controls without enzyme were also performed. The data obtained is shown in Fig. 6. This experiment does not take into account the melting temperature of the primer used in this experiment which at 60 °C would theoretically leave only a minority of the primer still being annealed to the template. This may actually result in an underestimate of the activity observed with the USB and Tth enzymes.

### Use of 64 kDa polymerase in Strand Displacement Amplification

The exonuclease deficient cloned enzyme described above was used in a Strand Displacement Amplification (SDA) reaction (Walker *et al*., (1992) *Proc. Natl. Acad. Sci. USA* 89:392-396). The target DNA to be amplified is a 1.2 kb clone of the insertion element IS 6110 from *Mycobacterium tuberculosis* [GenBank accession no. M29899]. The target DNA is cloned into pBluescript KS (Stratagene, La Jolla, CA). The reaction mixture (final concentrations: 25mM potassium phosphate, pH 7.4, 0.1 µg/mL BSA, 0.05 µM each primers B1 (CGATCGAGCAAGCCA) (SEQ. ID. NO. 16) and B2 (CGAGCCGCTCGCTGA) (SEQ. ID. NO. 17), 0.5 µM each primers S1.1 (ACCGCATCGAATGCATGTCTCGGTAGGCG ACTCGACC) (SEQ. ID. NO. 18) and S2.2 (CGATTCCGCTCAGACTTCTCGGGT TACTGAGATCCCT) (SEQ. ID. NO. 19), 1.4 mM dCTPαS, 0.5 mM each dATP, dGTP and dCTP and 10⁷ copies of target plasmid) was heated to 100 °C for 3 min to denature the target and then placed at 55 °C. After about 5 min at 55 °C, enzyme mix was added (final concentrations: 35 Units 64 kD polymerase, 160 Units *Bso*BI restriction enzyme (New England Biolabs, Beverly, MA) and 6.9 mM MgCl₂ (total reaction volume 50 µL) and incubation was continued for 10 min. An equal volume of stop solution (95% formamide, 20 mM EDTA and 0.05% each bromophenol blue and xylene cyanol FF) was added and 5 µL of this was electrophoresed on a 10% denaturing polyacrylamide gel. DNA was visualized by staining with ethidium bromide. The 64 kDa polymerase produced the expected 105 nucleotide full length product (major product) and the expected 86 nucleotide nicked product (minor product).

### Use of 64 kDa polymerase in RT-PCR

The 64 kD polymerase was used as a reverse transcriptase in conjunction with AmpliTaq in RT-PCR. Using rabbit globin mRNA as a template, 10⁶ copies were detectable under the following protocol. In a reaction volume of 100 µl, the following components were assembled: 1X PCR buffer (10 mM Tris-HCl, pH 8.3, 50 mM KCl), 2 mM MgCl₂, 200 µM each dNTP, 100 U human placental RNAse inhibitor (Amersham), 10⁶ copies rabbit globin mRNA (BRL), 30 pmoles globin-down primer (TCAGTGGTATTTGTGAGCCAGGGCATTGGCCACACCAGCCACCACCTTCT) (SEQ. ID. NO. 25), 15 pmole globin-up primer (ATCCCCCAAAACAGACAGAATGGTGCATCTGTCC) (SEQ. ID. NO. 26), 2.5 U AmpliTaq polymerase and 5 U 64 kD polymerase. The reaction was incubated at 65 °C for 30 min followed by 5 min at 95 °C, 35 cycles of (95 °C for 1 min, 65 °C for 1 min, 70 °C for 2 min) followed by 72 °C for 5 min. The product was resolved on a 2% ethidium bromide stained agarose gel and the expected 463 bp product was observed.

Using total RNA from rat liver, an RT-PCR product from the albumin gene was detected from 250 ng of template using the following protocol. In a reaction volume of 100 µL, the following components were assembled: 1X PCR buffer (10 mM Tris-HCl, pH 8.3, 50 mM KCl), 2 mM MgCl₂, 200 µM each dNTP, 100 U human placental RNAse inhibitor (Amersham), 250 ng rat liver RNA, 30 pmoles albumin-down primer (GTTCTCCTGGAAGGAGGTGCACATGGCCTC) (SEQ. ID. NO. 27), 15 pmole albumin-up primer (CACACAAGAGTGAGATCGCCCATCGGTTTAAGGA) (SEQ. ID. NO. 28), 2.5 U AmpliTaq polymerase and 40 U 64 kD polymerase. The reaction was incubated at 65 °C for 30 min followed by 5 min at 95 °C, 35 cycles of (95 °C for 1 min, 65 °C for 1 min, 70 °C for 2 min) followed by 72 °C for 5 min. The product was resolved on a 2% ethidium bromide stained agarose gel and the expected 386 bp product was observed.

### Example 9: Construction of FY DNA polymerase from Thermoanaerobacter thermohydrosulfuricus

### Starting Material

DNA constructs that were used to generate the FY polymerase were as follows:

pRED6706 is an expression vector which encodes a polypeptide of 608 amino acids. After an engineered initiating methionine, the remaining 607 amino acids encoded represent the C-terminal portion of the DNA polymerase I obtainable from of *Thermoanaerobacter thermohydrosulfuricus.* pRED6404 is an expression vector which encodes a polypeptide of 578 amino acids. After an engineered initiating methionine, the remaining 577 amino acids encoded represent the C-terminal portion of the DNA polymerase I obtainable from *Thermoanaerobacter thermohydrosulfuricus*.

pPREF2 encodes the same polypeptide as pRED6404, but has three silent mutations of the *Thermoanaerobacter thermohydrosulfuricus* sequence at codons 7, 10 and 11 to eliminate codons rarely utilized by *E. coli*.

All of these constructs are derived from expression vector pRE2 (*Nucleic Acids Res.* (1989) 17, 10473-88) which utilizes the λ P_{L} promoter and λ cII ribosome binding site.

### Construction of F412Y

*Thermoanaerobacter thermohydrosulfuricus* DNA polymerase was searched for the sequence (RXXXKXXXFXXXYG) (SEQ. ID. NO. 20) to locate the motif B or O-helix region. The sequence (RRAAKAVNFGIIYG) (SEQ. ID. NO. 21) was found at amino acid residues numbered 433 through 466 of the predicted pRED6706 gene product. A mutagenic oligonucleotide, DDFY(AAGGTCAGAAAGCCCATAATCGCTTATGCCATATATTATG CCATAATTTACGGC) (SEQ. ID. NO. 22) was designed anti parallel to nucleotides 1315-1371 of the pRED6706 coding sequence except for the change (AAA-ATA) at nt 1325. This change corresponds to phenylalanine being changed to tyrosine in the expressed polymerase. This oligonucleotide also overlaps a unique *Xho*l restriction site at nucleotide 1361.

Oligonucleotide DDFY together with DD64pref (SEQ. ID. NO. 13) which is complementary to nucleotides 94-133 of the pRED6706 coding sequence, contains an *Nde*I restriction site, were used to direct PCR amplification and simultaneous mutagenesis of pRED6706. The resulting PCR product was digested with *Nde*I and *Xho*I, purified by agarose gel electrophoresis and ligated with the large fragment of similarly treated pRED6404. The ligated construct was used to transform competent *E. coli* strain DH1 λ+ (*λcI* +). Clones yielding an appropriate restriction enzyme digestion pattern were sequenced to confirm the mutation. Spurious mutations were found in addition to the desired mutation in all clones examined, so an *Afl*III/*Kpn*I restriction fragment containing exclusively the F412Y mutation (pRED6404 numbering) was subcloned in pRED6404 to yield pLS-1.

### Example 10: Purification of F412Y DNA polymerase

Plasmid pLS-1 was transformed into *E. coli* strain MZ-1 (λ *cI*857 lysogen). One L of culture in LB medium containing 100 µg/mL Ampicillin was grown at 30 °C until OD₆₀₀ = 1.1. The temperature was raised to 42 °C and growth continued for 2 hours. The cells were harvested by centrifugation (4 g wet weight) and resuspended in 15 mL buffer A (50 mM Tris-HCl, pH 8.0, 1 mM DTT, 1 mM PMSF, 1 mM EDTA, 10% (v/v) glycerol) containing 50 mM NaCl. The resuspension was sonicated four times for 30 seconds and the lysate was cleared by centrifugation. The cleared lysate was made 300 mM in NaCl and passed through a column of DEAE cellulose (65 mL bed volume) equilibrated in buffer A containing 300 mM NaCl. The flow through fraction (45 mL) was collected, heated to 70 °C for 15 min and cleared by centrifugation. The cleared supernatant was diluted with buffer A containing 50 mM NaCl to a total volume of 120 mL. This solution was loaded onto a column of heparin sepharose (70 mL bed volume) equilibrated with buffer A containing 150 mM NaCl. The column was washed with this same buffer and the activity eluted from the column with a linear salt gradient from 150 mM to 700 mM NaCl in buffer A. Active fractions judged to be greater than 90% pure by SDS-PAGE were pooled, concentrated by ultrafiltration and dialyzed against 50 mM phosphate 50% (v/v) glycerol. The sequence is provided in Fig. 4A-C (SEQ. ID. NO. 4).

### Example 11: Full Length 5' to 3' Exonuclease Deficient Mutant

The removal of 5' to 3' exonuclease activity is desirable for applications suitable for this polymerase, including amplification and sequencing. Because the 5' to 3' exonuclease activity may be co-incident with RNaseH activity, it is also important to remove or reduce the 5' to 3' exonuclease activity for the application of reverse transcription. Single nucleotide substititions are suitable to reduce or remove exonuclease activity.

The amino acid sequence of Tts polymerase was compared with known homologous DNA Polymerase I enzymes such as *E. coli* DNA Pol I, Taq DNA polymerase and Tth DNA polymerase to identify conserved motifs in the 5' to 3' exonuclease domain (Gutman and Minton, Nucleic Acids Research, 21:4406-4407, 1993).

| | | | |
|---|---|---|---|
| Tts | 5 | LIIDGSS | 11 |
| Pol I | 10 | ILVDGSS | 16 |
| Taq | 15 | LLVDGHH | 21 |
| Tth | 15 | LLVDGHH | 21 |

A carboxylate residue known to be present at the exonuclease active site of Taq DNA polymerase (Kim et al., Nature 376:612-616, 1995) was mutangenized. This residue corresponds to aspartic acid residue at position 8 (D8) in Tts polymerase, which was mutagenized to an alanine residue (A) to remove the carboxylate function and abolish the 5' to 3' exonuclease activity. An oligo primer (CTTACATATGGCTTATAAATTTTTAATCATTGCTGGTAGTAG) (Seq. I.D. No. 28) was used to perform the site directed mutagenesis. This mutagenesis converts Asp⁸ to Ala⁸ and introduces a convenient cloning site (NdeI) at the 5' end. The pLS-3 construct (full length Tts polymerase) was used as template and parental clone. A 1.45 kb product was amplified by PCR with the above primer and an internal Tts reverse primer. The NdeI/EcoRI fragment of this PCR product was substituted back into the pLS-3 clone. Vector pRE2 and bacterial strains DH1 λ+ and M5248 are as described previously.

Those who practice the art can utilize known techniques, and others known to those who practice the art, to identify and alter other amino acid residues in the amino terminal one third of the Tts polymerase to remove the exonuclease activity. There are at least six conserved motifs located at the amino terminus of homologous DNA polymerases, in which changes in amino acid residues result in a loss or a reduction in exonuclease activity. These conserved motifs are identified in Gutman and Minton (supra) and are supported by data from the crystal structure of Taq polymerase (Kim, *et al*. (1995) *Nature* 376:612-616). The present invention encompasses alterations in these regions.

### Example 12: PCR with ΔTts in 30% Glycerol

PCR was performed with ΔTts enzyme in presence of 30% glycerol, in order to stabilize the enzyme and to reduce the temperatures necessary for DNA annealing in the reaction. A 100 µL PCR reaction contains 10 ng of a 6 kb plasmid template, 100 pmol each of forward and reverse primer about 500 bp apart, 200 µM dNTP, 10 mM Tris-HCL, pH 8.3, 50 mM KCL, 1.5 mM MgCL₂ and 30% glycerol. The reaction mix was heated to 95 °C for 2 min before ATts was added. An AmpliTaq™ (PE Applied Biosystems, Foster City, CA) control reaction was performed separately under the same conditions. The cycling program consisted of: 80 °C for 15 sec, 40 °C for 30 sec and 65 °C for 1 min, repeated for 30 cycles. A PCR product of appropriate size was generated by 2.5, 5 and 10 units of ΔTts, and this amount of product was two to three fold greater than that made by 2.5 units of AmpliTaq™ with the same conditions.

### Example 13: RT/PCR Protocol with ΔTts

Reverse transcription is performed with ΔTts enzyme, as the first step of RT/PCR. A 20 µL reaction contains 40 units of ΔTts enzyme, 5 mM MgCL₂, 20 units of placental Rnase inhibitor (Amersham Pharmacia Biotech, Inc., Cleveland, OH), 1 mM each of dATP, dCTP, dGTP, and dTTP, 15 pmol primer, 10 mM Tris-HCl pH 8.3, 50 mM KCl, and 50 ng globin mRNA template. The reaction is allowed to proceed for 45 min at 65 °C. This 20 µL reaction mixture is added to a PCR reaction mixture with a final volume of 100 µL, containing 10 mM Tris-HCl, pH 8.3, 50 mM KCl, 5 mM MgCL₂, 100 pmol each of forward and reverse primers about 500 bp apart, and 30% glycerol. Further addition of enzyme is not necessary; ΔTts performs PCR in the presence of glycerol. The cycling program consists of 50 cycles of: 80 °C for 15 sec, 40 °C for 30 sec, 65 °C for 1 min.

One skilled in the art would readily appreciate that the present invention is well adapted to carry out the objects and obtain the ends and advantages mentioned, as well as those inherent therein. The methods, kits, solutions, and molecules, described herein are presently representative of preferred embodiments are exemplary and are not intended as limitations on the scope of the invention. Changes therein and other uses will occur to those skilled in the art which are encompassed within the spirit of the invention are defined by the scope of the claims.

It will be readily apparent to one skilled in the art that varying substitutions and modifications may be made to the invention disclosed herein without departing from the scope and spirit of the invention.

All patents and publications mentioned in the specification are indicative of the levels of those skilled in the art to which the invention pertains. All patents and publications are herein incorporated by reference to the same extent as if each individual publication was specifically and individually indicated to be incorporated by reference.

The invention illustratively described herein suitably may be practiced in the absence of any element or elements, limitation or limitations which is not specifically disclosed herein. Thus, for example, in each instance herein any of the terms "comprising", "consisting essentially of" and "consisting of" may be replaced with either of the other two terms. The terms and expressions which have been employed are used as terms of description and not of limitation, and there is no intention that in the use of such terms and expressions of excluding any equivalents of the features shown and described or portions thereof, but it is recognized that various modifications are possible within the scope of the invention claimed.

In addition, where features or aspects of the invention are described in terms of Markush groups, those skilled in the art will recognize that the invention is also thereby described in terms of any individual member or subgroup of members of the Markush group.

Other embodiments are within the following claims.

## Claims

1. An enzymatically active DNA polymerase having at least 80% identity in its amino acid sequence to the DNA polymerase of *Thermoanaerobacter thermohydrosulfuricus* of Seq. ID. No. 2 and having an exonuclease activity removed by replacing the aspartic acid residue at position 8 with an alanine residue.

2. The DNA polymerase of claim 1, wherein said DNA polymerase has phenylalanine at position 706 replaced with tyrosine in Seq. ID. No. 2.

3. Kit for polymerase chain reaction comprising a polymerase stabilizing agent, and an enzymatically active DNA polymerase having at least 80% identity in its amino acid sequence to the DNA polymerase of *Thermoanaerobacter thermohydrosulfuricus* of Seq. ID. No. 2 and an exonuclease activity removed by replacing the aspartic acid residue at position 8 with an alanine.

4. The kit of claim 3 which also contains an agent independently selected from the group consisting of glycerol, trimethylamine-N-oxide, and N-methylmorpholine-N-oxide.

5. Method for polymerase chain reaction or reverse transcription, in the presence of a polymerase stabilizing agent, utilizing an enzymatically active DNA polymerase having at least 80% identity in its amino acid sequence to the DNA polymerase of *Thermoanaerobacter thermohydrosulfuricus* of Seq. ID. No. 2 and an exonuclease activity removed by replacing the aspartic acid residue at position 8 with an alanine.

6. The method of claim 5, wherein said agent is independently selected from the group consisting of glycerol, trimethylamine-N-oxide, and N-methylmorpholine-N-oxide.

7. Method for generating and amplifying a nucleic acid fragment by strand displacement utilizing a DNA polymerase having at least 80% identity in its amino acid sequence to the DNA polymerase of *Thermoanaerobacter thermohydrosulfuricus* of Seq. ID. No. 2 and having an exonuclease activity removed by replacing the aspartic acid residue at position 8 with an alanine residue.

8. Method for sequencing DNA whereby at least a part of the nucleotide base sequence of a DNA template can be determined comprising the step of generating chain terminated fragments from said DNA template with a DNA polymerase of claim 1 in the presence of at least one chain terminating agent and determining the sequence of at least part of said DNA template from the sizes of said fragments.

9. Kit for sequencing DNA comprising a DNA polymerase of claim 1 and a pyrophosphatase.

## Patentansprüche

1. Enzymatisch aktive DNA-Polymerase, die mindestens 80% Identität in ihrer Aminosäuresequenz zur DNA-Polymerase von Thermoanaerobacter thermohydrosulfuricus der Seq. ID. Nr. 2 besitzt und deren Exonuclease-Aktivität durch Austauschen des Asparaginsäure-Restes in Position 8 mit einem Alanin-Rest entfernt ist.

2. DNA-Polymerase nach Anspruch 1, bei der Phenylalanin in Position 706 mit Tyrosin in Seq. ID. Nr. 2 ausgetauscht ist.

3. Kit für eine Polymerasekettenreaktion, umfassend ein Polymerasestabilisationsmittel und eine enzymatisch aktive DNA-Polymerase, die mindestens 80% Identität in ihrer Aminosäuresequenz zur DNA-Polymerase von Thermoanaerobacter thermohydrosulfuricus der Seq. ID. Nr. 2 besitzt und deren Exonuclease-Aktivität durch Austauschen des Asparaginsäure-Restes in Position 8 mit einem Alanin entfernt ist.

4. Kit nach Anspruch 3, das auch ein Mittel enthält, das unabhängig ausgewählt ist aus der Gruppe, die besteht aus Glycerol, Trimethylamin-N-oxid und N-Methylmorpholin-N-oxid.

5. Verfahren zur Polymerasekettenreaktion oder Reversen Transkription, in Anwesenheit eines Polymerasestabilisationsmittels, unter Verwenden einer enzymatisch aktiven DNA-Polymerase, die mindestens 80% Identität in ihrer Aminosäuresequenz zur DNA-Polymerase von Thermoanaerobacter thermohydrosulfuricus der Seq. ID. Nr. 2 besitzt und deren Exonudease-Aktivität durch Austauschen des Asparaginsäure-Restes in Position 8 mit einem Alanin entfernt ist.

6. Verfahren nach Anspruch 5, bei dem das Mittel unabhängig ausgewählt ist aus der Gruppe, die besteht aus Glycerol, Trimethylamin-N-oxid und N-Methylmorpholin-N-oxid.

7. Verfahren zum Erzeugen und Amplifizieren eines Nucleinsäurefragmentes durch Strangverdrängung unter Verwenden einer DNA-Polymerase, die mindestens 80% Identität in ihrer Aminosäuresequenz zur DNA-Polymerase von Thermoanaerobacter thermohydrosulfuricus der Seq. ID. Nr. 2 besitzt und deren Exonuklease-Aktivität durch Austauschen des Asparaginsäure-Restes in Position 8 mit einem Alanin-Rest entfernt ist.

8. Verfahren zum Sequenzieren von DNA, wodurch mindestens ein Teil der Nucleotidbasensequenz einer DNA-Matrize bestimmt werden kann, umfassend den Schritt des Erzeugens von Ketten-terminierten Fragmenten aus der DNA-Matrize mit einer DNA-Polymerase des Anspruchs 1 in Anwesenheit mindestens eines Ketten-terminierenden Mittels und das Bestimmen der Sequenz mindestens eines Teils der DNA-Matrize aus den Größen der Fragmente.

9. Kit zum Sequenzieren von DNA, umfassend eine DNA-Polymerase nach Anspruch 1 und eine Pyrophosphatase.

## Revendications

1. Une ADN polymérase active de manière enzymatique ayant au moins 80% d'identité dans sa séquence d'acides aminés avec l'ADN polymérase de séquence Seq. ID. No. 2 de *Thermoanaerobacter thermohydrosulfuricus* et ayant une activité exonucléase enlevée en remplaçant le résidu d'acide aspartique à la position 8 par un résidu d'alanine.

2. L'ADN polymérase de la revendication 1, dans laquelle ladite ADN polymérase de séquence Seq. ID. No. 2 a une phénylalanine en position 706 remplacée par une tyrosine.

3. Un kit pour la réaction en chaîne de polymérase comportant un agent stabilisant de polymérase, et une ADN polymérase active de manière enzymatique ayant au moins 80% d'identité dans sa séquence d'acides aminés avec l'ADN polymérase de séquence Seq. ID. No. 2 de *Thermoanaerobacter thermohydrosulfuricus* et une activité exonucléase enlevée en remplaçant le résidu d'acide aspartique à la position 8 par une alanine.

4. Le kit de la revendication 3 qui contient également un agent indépendamment choisi dans le groupe constitué de glycérol, de triméthylamine-N-oxyde, et de N-methylmorpholine-N-oxyde.

5. Un procédé pour la réaction en chaîne ou la transcription inverse de polymérase, en présence d'un agent stabilisant de polymérase, utilisant une ADN polymérase active de manière enzymatique ayant au moins 80% d'identité dans sa séquence d'acides aminés avec l'ADN polymérase de séquence Seq. ID. No. 2 de *Thermoanaerobacter thermohydrosulfuricus* et une activité exonucléase enlevée en remplaçant le résidu d'acide aspartique à la position 8 par une alanine.

6. Le procédé de la revendication 5, dans lequel ledit agent est indépendamment choisi dans le groupe constitué de glycérol, de triméthylamine-N-oxyde, et de N-methylmorpholine-N-oxyde.

7. Un procédé pour produire et amplifier un fragment d'acide nucléique par déplacement de brin en utilisant une ADN polymérase ayant au moins 80% d'identité dans sa séquence d'acides aminés avec l'ADN polymérase de séquence Seq. ID. No. 2 de *Thermoanaerobacter thermohydrosulfuricus* et une activité exonucléase enlevée en remplaçant le résidu d'acide aspartique à la position 8 par un résidu d'alanine.

8. Un procédé pour séquencer l'ADN, par lequel au moins une partie de la séquence de base de nucléotides d'une matrice d'ADN peut être déterminée, comportant l'étape de production de fragments fin de chaîne à partir de ladite matrice d'ADN avec une ADN polymérase de la revendication 1 en présence d'au moins un agent fin de chaîne et de détermination de la séquence d'au moins une partie de ladite matrice d'ADN à partir des tailles desdits fragments.

9. Un kit pour séquencer l'ADN comportant une ADN polymérase de la revendication 1 et une pyrophosphatase.
